# EUROPÄISCHE PATENTSCHRIFT

(11) **EP 1 684 729 B1**
(45) Veröffentlichungstag und Bekanntmachung des Hinweises auf die Patenterteilung: **14.05.2008**
(21) Anmeldenummer: 04765211.0
(22) Anmeldetag: 15.09.2004
(51) Int. Cl.: A61K 9/20, A61K 9/28

(54) **MEHRSCHICHTIGE ARZNEIFORM, ENTHALTEND EINE IN BEZUG AUF DIE WIRKSTOFFREIGABE MODULARISCH WIRKENDE SUBSTANZ**
MULTILAYER PHARMACEUTICAL DOSAGE FORM CONTAINING A SUBSTANCE THAT ACTS IN A MODULATORY MANNER WITH REGARD TO THE RELEASE OF ACTIVE SUBSTANCES
FORME GALENIQUE MULTICOUCHE CONTENANT UNE SUBSTANCE A ACTION MODULATRICE PAR RAPPORT A LA LIBERATION DU PRINCIPE ACTIF

(30) Priorität: 13.11.2003 DE 10353186
(43) Veröffentlichungstag der Anmeldung: 02.08.2006
(73) Patentinhaber: Evonik Röhm GmbH, 64293 Darmstadt (DE)
(72) Erfinder: LIZIO, Rosario, 64380 Rossdorf (DE); PETEREIT, Hans-Ulrich, 64291 Darmstadt (DE); ASSMUS, Manfred, 64404 Bickenbach (DE); RAVISHANKAR, Hema, Chembur, Mumbai 400089 (IN)
(74) Vertreter: Gottschalk, Michael
(86) Internationale Anmeldenummer: PCT/EP2004/010297
(87) Internationale Veröffentlichungsnummer: WO 2005/046649

(56) Entgegenhaltungen:
- EP-A- 1 213 015
- DE-A1- 19 956 486
- US-B1- 6 322 819

## Beschreibung

Die Erfindung betrifft eine mehrschichtige Arzneiform für die kontrollierte Wirkstofffreisetzung.

### Stand der Technik

EP-A 0 463 877 beschreibt pharmazeutische Zusammensetzungen mit verzögerter Wirkstofffreisetzung, bestehend aus einem Kern mit einem pharmazeutischen Wirkstoff und einem einschichtigen Überzugsfilm der ein wasserabweisendes Salz und ein wasserunlösliches Copolymer aus Ethylacrylat, Methylmethacrylat und Trimethylammoniumethlymethacrylat-Chlorid enthält. Das wasserabweisende Salz kann z. B. Ca- oder Mg-Stearat sein. Es werden sigmoide Freisetzungskurven erhalten.

EP-A 0 225 085, EP-A 0 122 077 und EP-A 0 123 470 beschreiben die Verwendung organischer Säure in Arzneimittelkernen, die mit verschiedenen Überzügen aus organischen Lösungen versehen werden. Es resultieren im wesentlichen sigmoide Freisetzungscharakteristiken.

EP-A 0 436 370 beschreibt pharmazeutische Zusammensetzungen mit verzögerter Wirkstofffreisetzung, bestehend aus einem Kern mit einem pharmazeutischen Wirkstoff und einer organischen Säure und einem äußeren Überzugsfilm der durch wäßriges Sprühen aufgebracht wurde und ein Copolymer aus Ethylacrylat, Methylmethacrylat und Trimethylammoniumethlymethacrylat-Chlorid ist. Dabei werden ebenfalls sigmoide Freisetzungskurven erhalten.

WO 00/19984 beschreibt eine pharmazeutische Zubereitung bestehend aus (a) einem Kern, enthaltend einen Wirkstoff, gegebenenfalls einen Träger und übliche pharmazeutische Zusatzstoffe, sowie das Salz einer organischen Säure, dessen Anteil am Kerngewicht 2,5 bis 97,5 Gew.-% ausmacht und (b) einem äußeren Überzugsfilm, der aus einem oder mehreren (Meth)acrylat-Copolymeren sowie gegebenenfalls aus üblichen pharmazeutischen Hilfsstoffen besteht, wobei 40 bis 100 Gew.-% der (Meth)acrylat-Copolymeren zu 93 bis 98 Gew.-% aus radikalisch polymerisierten C₁- bis C₄-Alkylestern der Acryl- oder der Methacrylsäure und 7 bis 2 Gew.-% (Meth)acrylat-Monomeren mit einer quaternären Ammoniumgruppe im Alkylrest bestehen und gegebenenfalls in einer Mischung vorliegen können mit 1 bis 60 Gew.-% einem oder mehreren weiteren, vom erstgenannten (Meth)acrylat-Copolymeren verschiedenen (Meth)acrylat-Copolymeren, die sich zu 85 bis 100 Gew.-% aus radikalisch polymerisierten C₁- bis C₄-Alkylestern der Acryl- oder der Methacrylsäure und gegebenenfalls bis zu 15 Gew.-% weiterer (Meth)acrylat-Monomere mit basischen Gruppen oder Säuregruppe im Alkylrest zusammensetzen.

WO 00/74655 beschreibt ein Wirkstofffreigabesystem mit zweifachem Freigabe-Puls, der durch einen dreischichtigem Aufbau bewirkt wird. Der Kern enthält einen Wirkstoff und eine in Gegenwart von Wasser quellende Substanz, z. B. eine vernetzte Polyacrylsäure. Ein innerer Überzug besteht aus einem wasserunlöslichen Trägermaterial, z. B. einem kationischen (Meth)acrylat-Copolymer, und enthält ein wasserlösliches partikulares Material, z. B. ein Pektin, wodurch eine Porenbildung erreicht werden kann. Ein äußerer Überzug enthält den gleichen oder einen anderem Wirkstoff. Im Gastrointestinaltrakt wird zunächst der außen liegende Wirkstoff freigesetzt, während der im Kern vorhandene Wirkstoff zeitlich versetzt durch die Poren in der mittleren Schicht freigegeben wird. Die dreischichtige Arzneiform kann optional noch einen weiteren Überzug, z. B. aus einem Carboxylgruppen-haltigen (Meth)acrylat-Copolymeren aufweisen.

US 5,508,040 beschreibt eine multipartikuläre Arzneiform, die aus einer Vielzahl von Pellets besteht, die in einen Bindemittel zusammengehalten werden. Die Pellets weisen im Kern einen Wirkstoff und einen osmotisch aktiven Modulator, z. B. NaCl oder eine organische Säure, auf. Die Pelletkerne werden mit unterschiedlich dicken Überzügen, z. B. aus (Meth)acrylat-Copolymeren mit quaternären Ammoniumgruppen, versehen. Zur Reduzierung der Permeabilität enthalten die Überzüge noch hydrophobe Substanzen, z. B. Fettsäuren, in Mengen von 25 Gew.-% oder darüber. Die multipartikuläre Arzneiform setzt sich durch eine den enthaltenen Wirkstoff in einer Vielzahl von Pulsen frei, die der Anzahl der unterschiedlich dick überzogenen Pelletpopulationen entspricht.

EP 1 064 938 A1 beschreibt eine Arzneiform, die im Kern einen Wirkstoff und ein oberflächenaktive Substanz (Surfactant) aufweist. Der Kern kann zusätzlich eine organische Säure enthalten und ist überzogen mit (Meth)acrylat-Copolymeren mit quaternären Ammoniumgruppen. Es werden "gepulste" Freigabekurven erhalten. Treppenartige Freigabekurven können durch die Kombination unterschiedlich überzogener Pellets in einer Arzneiform erhallen werden.

WO 01/13895 beschreibt bimodale Freigabesysteme für Wirkstoffe mit sedativ hypnotischer Wirkung. Die Freigabeprofile werden durch Mischungen verschiedener Pelletpopulationen realisiert.

WO 01/37815 beschreibt mehrschichtige Freigabesysteme zur gesteuerten, pulsartigen Abgabe von Wirkstoffen. Dabei ist eine innere Membran vorhanden, die durch die im Kern vorhandene Wirkstoffformulierung aufgelöst werden kann.

Weiterhin ist eine äußere Membran vorhanden, die zusätzlich eine poren bildende Substanz aufweist.

WO 01/58433 beschreibt mehrschichtige Freigabesysteme zur gesteuerten, pulsartigen Abgabe von Wirkstoffen. Dabei ist der Wirkstoff im Kern enthalten und wird von einer darmsaftlöslichen Polymermembran umgeben. Eine äußere Membran besteht aus einer Mischung eines darmsaftlöslichen Polymers mit einem wasserunlöslichen Polymer in definierten Mengenbereichen. Zwischen der inneren und der äußeren Membran kann sich eine Zwischenschicht befinden, die eine organische Säure enthält.

### Aufgabe und Lösung

Ausgehend von EP-A 0 436 370 und WO 00/19984 sollte eine Arzneiform entwickelt werden, die es erlaubt die Permeabilität von Filmüberzügen durch intrinsische Modulation zu beeinflussen, so dass Freigabeprofile mit Verläufen nullter Ordnung, erster Ordnung, erster Ordnung mit einsetzender Beschleunigungsphase, langsam-schnell, schnell-langsam individuell je nach Wirkstoff und therapeutischen Bedarf eingestellt werden können.

Die Aufgabe wird gelöst durch eine
Mehrschichtige Arzneiform für die kontrollierte Wirkstofffreisetzung, enthaltend
a) eine Kernschicht, enthaltend eine in Bezug auf die Wirkstoffabgabe modulatorisch wirkende Substanz, gegebenenfalls einen neutralen Kern und/oder einen Wirkstoff,
b) eine innere Kontrollschicht, die die Abgabe der modulatorisch wirkenden Substanz und des gegebenenfalls enthaltenen Wirkstoffs aus der Kernschicht beeinflusst, bestehend aus pharmazeutisch verwendbaren Polymeren, Wachsen, Harze und/oder Proteinen
c) eine Wirkstoffschicht, enthaltend einen pharmazeutischen Wirkstoff und gegebenenfalls eine modulatorisch wirkende Substanz,
d) eine äußere Kontrollschicht, enthaltend zu mindestens 60 Gew.-% ein oder eine Mischung aus mehreren (Meth)acrylatcopolymeren, aus 98 bis 85 C₁- bis C₄-Alkylestern der (Meth)acrylsäure und 2 bis 15 Gew.-% Methacrylatmonomeren mit einer quaternären Ammoniumgruppe im Alkylrest, und gegebenenfalls bis zu 40 Gew.-% weitere pharmazeutisch verwendbare Polymere,
wobei die Schichten zusätzlich und in an sich bekannter Weise pharmazeutisch übliche Hilfsstoffe enthalten können.

### Ausführung der Erfindung

Die Erfindung betrifft eine mehrschichtige Arzneiform für die kontrollierte Wirkstofffreisetzung, enthaltend im wesentlichen, eine Kernschicht a) und die Schichten b), c) und d). Zusätzlich können noch übliche Topcoatschichten, die z. B. pigmentiert sein können, vorhanden sein.

### Die Kernschicht a)

Die mehrschichtige Arzneiform besitzt eine Kernschicht a), enthaltend eine in Bezug auf die Wirkstoffabgabe modulatorisch wirkende Substanz, gegebenenfalls einen neutralen Kern (Nonpareilles) und/oder einen Wirkstoff.

Geeignete Herstellungsverfahren für die Kernschicht a) sind direktes Verpressen, Verpressen von Trocken-, Feucht- oder Sintergranulaten, Extrusion und anschließende Ausrundung, feuchte oder trockene Granulation oder direkte Pelletierung (z.B. auf Tellern) oder durch Binden von Pulvern (Powder layering) auf wirkstofffreie Kugeln bzw. Kerne (Nonpareilles) oder wirkstoffhaltige Partikeln.
Neben dem Wirkstoff, der in Bezug auf die Wirkstoffabgabe modulatorisch wirkenden Substanz und dem gegebenenfalls vorhandenen neutralen Kern (Nonpareilles) kann die Kernschicht a) weitere pharmazeutische Hilfsstoffe enthalten: Bindemittel, wie Zellulose und deren Derivate, Polyvinylpyrrolidon (PVP), Feuchthaltemittel, Zerfallsförderer, Gleitmittel, Sprengmittel, Stärke und deren Derivate, Zucker Solubilisatoren oder andere.

### Alternativen für den Aufbau der Kernschicht a)

Die Kernschicht kann alternativ und im wesentlichen folgende Bestandteile enthalten
I. eine modulatorisch wirkende Substanz z. B. in Kristall-, Granulat-, oder Copräzipitatform. Die Größe von Granulaten oder Kristallen kann z. B. zwischen 0,01 und 2,5 mm, liegen,
II. eine modulatorisch wirkende Substanz und einen Wirkstoff, die in beliebiger Reihenfolge schichtweise aufeinander oder in Mischung vorliegen können,
III. einen neutralen Kern (Nonpareilles), beschichtet mit einer modulatorisch wirkenden Substanz,
IV. einen neutralen Kern (Nonpareilles), beschichtet mit einer modulatorisch wirkende Substanz und einem Wirkstoff, die in beliebiger Reihenfolge schichtweise aufeinander oder in Mischung vorliegen können.

### Modulatorisch wirkende Substanzen

Erfindungsgemäß zu verwendende modulatorisch wirkende Substanz können ein Molekulargewicht unter 500 aufweisen, in fester Form vorliegen und ionogen sein.

Bevorzugt ist die modulatorisch wirkende Substanz wasserlöslich.

Die modulatorisch wirkende Substanz kann z. B. eine organische Säure oder das Salz einer organischen oder anorganischen Säure sein.

Die modulatorisch wirkende Substanz kann z. B. Bernsteinsäure, Citronensäure, Weinsäure, Laurylschwefelsäure, ein Salz dieser Säuren oder ein Salz aus folgenden Anionen sein: Taurochlolat und andere Cholate, Chloride, Acetate, Lactate, Phosphate und/oder Sulfate.

### Funktionsweise der Komponenten untereinander

Die Funktionsweise der modulatorisch wirkenden Substanz in der mehrschichtigen Arzneiform kann in etwa wie folgt beschrieben werden:
Na-Succinat (Bernsteinsäure), Na-Acetat und Zitronensäure beschleunigen die Wirkstoffabgabe.
NaCl und Na-Citrat verlangsamen die Wirkstoffabgabe.

Enthält die Wirkstoffschicht c) zusätzlich zur inneren Kernschicht a) eine modulatorisch wirkende Substanz, so bestimmt sich die Wirkstoffabgabe zunächst durch die in der äußeren Schicht, der Wirkstoffschicht c), enthaltene modulatorisch wirkenden Substanz. Ist diese weitgehend verbraucht, setzt die Wirkung der modulatorisch wirkenden Substanz in der inneren Schicht, der inneren Kernschicht a), ein und bestimmt die weitere Wirkstoffabgabe.

Durch die Kombination unterschiedlicher Mengen einer und/oder verschiedener modulatorisch wirkender Substanzen in beiden Schichten lassen sich die verschiedenen Wirkstoffabgabeprofile an den Wirkstoff bzw. das therapeutische Ziel anpassen. Hinzu kommt noch die Wirkung der inneren Kontrollschicht b), die ihrerseits wiederum die Abgabe der modulatorisch wirkenden Substanz aus der Kemschicht a) steuert.

Die Menge der Wirkstoffabgabe wird im wesentlichen durch die äußere Kontrollschicht d) gesteuert. Enthält die innere Kontrollschicht zusätzlich einen Wirkstoff, kann diese zur Justierung des Wirkstoffabgabeprofils gegen Ende der Wirkstoffabgabe genutzt werden.

Enthalten die Wirkstoffe selbst ionische Gruppen bzw. liegen in der Salzform vor, so kann der Wirkstoff selbst die Wirkung der modulatorisch wirkenden Substanz bzw. Substanzen dahingehend beeinfussen, daß diese abgeschwächt oder verstärkt wird. Diese Interaktion kann als weiteres Steuerungselement genutzt werden. Die ist z. B. bei den Wirkstoffen Metoprolol-Succinat und Terbutalin-Sulfat der Fall.

### Die innere Kontrollschicht b)

Die innere Kontrollschicht, beeinflusst die Abgabe der modulatorisch wirkenden Substanz und des gegebenenfalls enthaltenen Wirkstoffs aus der Kernschicht. Die innere Kontrollschicht enthält im wesentlichen pharmazeutisch verwendbare Polymere, Wachse und/oder Proteine. Zur Unterstützung der Formulierung können weitere pharmazeutisch übliche Hilfsstoffe wie z. B. Bindemittel, wie Zellulose und deren Derivate, Weichmacher, Polyvinylpyrrolidon (PVP), Feuchthaltemittel, Zerfallsförderer, Gleitmittel, Sprengmittel, Stärke und deren Derivate, Zucker und/oder Solubilisatoren beigemengt sein.

Die innere Kontrollschicht b) kann z. B. aus einem Polymeren bestehen, das wasserunlöslich oder in Wasser nur quellbar ist.

Geeignet sind z. B. die folgenden Polymere:
Copolymere aus Methylmethacrylat und/oder Ethylacrylat und Methacrylsäure, Copolymere aus Methylmethacrylat, Methylacrylat und Methacrylsäure, Copolymere aus Methylmethacrylat, Butylmethacrylat und Dimethylethylmethacrylat, Copolymere aus Methylmethacrylat, Ethylacrylat und Trimethylammoniumethylmethacrylat, Copolymere aus Methylmethacrylat und Ethylacrylat, Copolymere aus Ethylacrylat, Methylacrylat, Butylmethacrylat und Methacrylsäure
Polyvinylpyrolidone (PVP), Polyvinylalkohole, Polyvinylalkohol-Polyethylenglycol-Graft-Copolymer (Kollicoat®), Stärke und deren Derivate, Polyvinylacetatphtalat (PVAP, Coateric®), Polyvinylacetat (PVAc, Kollicoat), Vinylacetat-Vinylpyrolidon-Copolymer (Kollidon® VA64), Vinylacetat : Crotonsäure-Copolymer 9:1 (VAC : CRA, Kollicoat® VAC), Polyethylenglykole mit einem Molekulargewicht über 1000 (g/mol), Chitosan, ein (Meth)acrylatcopolymer, bestehend aus 20 - 40 Gew.-% Methylmethacrylat und 60 bis 80 Gew.-% Methacrylsäure, eine vernetzte und/oder unvernetzte Polyacrylsäure, ein Na-Alginat, und/oder ein Pektin,
Cellulosen wie z. B. anionische Carboxymethylcellulose und deren Salze (CMC, Na-CMC, Ca-CMC, Blanose, Tylopur), Carboxymethylethyldellulose (CMEC, Duodcell®), Hydroxyethylcellulose (HEC, Klucel), Hydroxypropylcellulose (HPC), Hydroxypropylmethylcellulose (HPMC, Pharmacoat, Methocel, Sepifilm, Viscontran, Opadry,), Hydroxymrthylethylcellulose (HEMC), Ethylcellulose (EC, Ethocel^{®}, Aquacoat^{®}, Surelease^{®}), Methylcellulose (MC, Viscontran, Tylopur, Methocel), Celluloseester, Celluloseglycolat, Celluloseacetatphtalat (CAP, Cellulosi acetas, PhEur, Celluloseacetate-phtalate, NF, Aquateric®), Celluloseacetatsuccinat (CAS), Celluloseacetattrimeliat (CAT), Hydroxypropylmethylcellulosephtalat (HPMCP, HP50, HP55), Hydroxypropylmethylcelluloseacetatsuccinat (HPMCAS -LF, -MF, -HF).

Die innere Kontrollschicht kann aus einem Wachs, wie z. B. Carnaubawachs, und/oder Bienenwachs bestehen bzw. dieses enthalten.

Die innere Kontrollschicht kann das Harz Shellack enthalten bzw. aus diesem bestehen.

Die innere Kontrollschicht kann ein Protein, wie z. B. Albumin, Gelatine, Zein, Gluten, Kollagen und/oder Lektine enthalten bzw. daraus bestehen. Das Protein der inneren Kontrollschicht soll bevorzugter Weise keine therapeutische Funktion, wie dies bei Protein- oder Peptidwirkstoffen der Fall ist, aufweisen, damit sich die technischen Effekte der inneren Kontrollschicht b) auf der einen Seite und der Wirkstoffschicht c) bzw. der Kernschichtschicht a), sofern diese einen Wirkstoff enthält, auf der anderen Seite nach Möglichkeit nicht überlagern.

### Die Wirkstoffschicht c)

Die Wirkstoffschicht c), enthält einen pharmazeutischen Wirkstoff, der mit dem Wirkstoff der Kernschicht identisch oder verschieden sein kann, sowie gegebenenfalls eine modulatorisch wirkende Substanz, die mit der modulatorisch wirkende Substanz der Kernschicht identisch oder verschieden sein kann.

### Wirkstoffe

Die erfindungsgemäße mehrschichtige Arzneiform ist im Prinzip für beliebige Wirkstoffe geeignet. Gebräuchliche Arzneistoffe sind in Nachschlagewerken, wie z.B. der Roten Liste oder dem Merck Index zu entnehmen.

Die im Sinne der Erfindung eingesetzten Arzneistoffe sind dazu bestimmt, am oder im menschlichen oder tierischen Körper Anwendung zu finden, um
1. Krankheiten, Leiden, Körperschäden oder krankhafte Beschwerden zu heilen, zu lindern, zu verhüten oder zu erkennen.
2. die Beschaffenheit, den Zustand oder die Funktionen des Körpers oder seelische Zustände erkennen lassen.
3. vom menschlichen oder tierischen Körper erzeugte Wirkstoffe oder Körperflüssigkeiten zu ersetzen.
4. Krankheitserreger, Parasiten oder körperfremde Stoffe abzuwehren, zu beseitigen oder unschädlich zu machen oder
5. die Beschaffenheit, den Zustand oder die Funktionen des Körpers oder seelische Zustände zu beeinflussen.

Die erfindungsgemäße Formulierung eignet sich zur Verabreichung grundsätzlich beliebiger pharmazeutischer Wirkstoffe oder biologisch aktiver Substanzen, die vorzugsweise als Bestandteil einer multipartikulären Arzneiform, von pellethaltigen Tabletten, Minitabletten, Kapseln, Sachets, Brausetabletten oder Trockensäften verabreicht werden können.

### Therapieklassen

Diese pharmazeutisch aktiven Substanzen können einer oder mehrerer Wirkstoffklassen angehören, wie ACE-Hemmer, Adrenergika, Adrenocortikosteroide, Aknetherapeutika, Aldose-Reduktase-Hemmer, Aldosteron-Antagonisten, Alpha-Glucosidasehemmer, Alpha 1- Antagonisten, Mittel gegen Alkoholabusus, Aminosäuren, Amöbizide, Anabolika, Analeptika, Anaesthetika-Zusätze, Anaesthetika (nicht inhalativ), Anaesthetika (lokal), Analgetika, Androgene, Anginatherapeutika, Antagonisten, Antiallergika, Antiallergika wie PDE-Hemmer, Antiallergika zur Asthmabehandlung, Weitere Antiallergika (z.B. Leukotrienantagonisten, Antianämika, Antiandrogene, Antianxiolytika, Antiarthritika, Antiarrhythmika, Antiatheriosklerotika, Antibiotika, Anticholinergika, Anticonvulsiva, Antidepressiva, Antidiabetika, Antidiarrhoika, Antidiuretika, Antidots, Antiemetika, Antiepileptika, Antifibrinolytika, Antiepileptika, Antihelmintika, Antihistaminika, Antihypotensiva, Antihypertensiva, Antihypertonika, Antihypotonika, Antikoagulantien, Antimykotika, Antiöstrogene, Antiöstrogene (Nicht-Steroide), Antiparkinson-Mittel, Antiphlogistika, Antiproliferative Wirkstoffe, Antiprotozoen Wirkstoffe, Antirheumatika, Antischistosomizide, Antispasmolytika, Antithrombotika, Antitussiva, Appetitzügler, Arteriosklerosemittel, Bakteriostatika, Betablocker, Betarezeptorenblocker, Bronchodilatoren, Carboanhydrase-Hemmer, Chemotherapeutika, Choleretika, Cholinergika, Cholinergische Agonisten, Cholinesterase-Hemmer, Mittel zur Behandlung von Colitis ulcerosa, Cyclooxigenasehemmer, Diuretika, Ektoparasitizide, Emetika, Enzyme, Enzym-Hemmer, Enzyminhibitoren, Wirkstoffe gegen Erbrechen, Fibrinolytika, Fungistatika, Gichtmittel, Glaukomtherapeutika, Glucocorticoide, Glucocortikosteroide, Hämostatika, Herzglykoside, Histamin H2-Antagonisten, Hormone und deren Hemmstoffe, Immuntherapeutika, Kardiotonika, Kokkidiostatika, Laxantien, Lipidsenker, Magen-Darmtherapeutika, Malariatherapeutika, Migränemittel, Mikrobiozide, Morbus Crohn, Metastasenhemmer, Migränemittel, Mineralstoffpräparate, motilitätssteigernde Wirkstoffe, Muskelrelaxantien, Neuroleptika, Wirkstoffe zur Behandlung der Oestrogene, Osteoporose, Otologika, Parkinsonmittel, Phytopharmaka, Protonenpumpenhemmer, Prostaglandine, Wirkstoffe zur Behandlung der benignen Prostatahyperblasie, Wirkstoffe zur Behandlung des Pruritus, Psoriasis Wirkstoffe, Psychopharmaka, Radikalfänger, Renin-Antagonisten, Schilddrüsentherapeutika, Wirkstoffe zur Behandlung von Seborrhoe, Wirkstoffe gegen Seekrankheit, Spasmolytika, alpha- und beta-Sympatomimetika, Tenatoprazol, Thrombozytenaggregationshemmer, Tyrosinkinaseinhibitoren, Tranquilizer, Ulkustherapeutika, Weitere Ulkustherapeutika, Mittel zur Behandlung der Urolithiasis, Virustatika, Vitamine, Zytokine, Wirkstoffe für die Kombinationstherapie mit Zytostatika, Zytostatika.

### Wirkstoffe

Beispiele geeigneter Wirkstoffe sind Acarbose, Acetylsalicylsäure, Abacavir, Aceclofenac, Aclarubicin, Acyclovir, Actinomycin, Adalimumab, Adefovir, Adefovirdipivoxil, Adenosylmethionin, Adrenalin und Adrenalinderivate, Agalsidase alpha, Agalsidase beta, Alemtuzumab, Almotriptan, Alphacept, Allopurinol, Almotriptan, Alosetron, Alprostadil, Amantadin, Ambroxol, Amisulprid, Amlodipin, Amoxicillin, 5-Aminosalicylsäure, Amitriptylin, Amlodipin, Amoxicillin, Amprenavir, Anakinra, Anastrozol, Androgen und Androgenderivate, Apomorphin, Aripiprazol, Arsentrioxid, Artemether, Atenolol, Atorvastatin, Atosiban, Azathioprin, Azelainsäure, Barbitursäurederivate, Balsalazid, Basiliximab, Beclapermin, Beclomethason, Bemiparin, Benzodiazepine, Betahistin, Bexaroten. Bezafibrat, Bicalutamid, Bimatoprost, Bosentan, Botulinumtoxim, Brimonidin, Brinzolamid, Budesonid, Budipin, Bufexamac, Bumetanid, Buprenorphin, Bupropion, Butizin, Calcitonin, Calciumantagonisten, Calciumsalze, Candesartan, Capecitabin, Captopril, Carbamazepin, Carifenacin, Carvedilol, Caspofungin, Cefaclor, Cefadroxil, Cefalexin Cefalosporine, Cefditoren, Cefprozil, Celecoxib, Cepecitabin, Cerivastatim, Cetirizin, Cetrorelix, Cetuximab, Chenodeoxycholsäure, Choriogonadotropin, Ciclosporin, Cidofovir, Cimetidin, Ciprofloxacin, Cisplatin, Cladribin, Clarithromycin, Clavulansäure, Clindamycin, Clobutinol, Clonidin, Clopidogrel, Codein, Coffein, Colestyramin, Cromoglicinsäure, Cotrimoxazol, Cumarin und Cumarinderivate, Darbepoetin, Cysteamin, Cystein, Cytarabin, Cyclophosphamid, Cyproteron, Cytarabin, Daclizumab, Dalfopristin, Danaparoid, Dapiprazol, Darbepoetin, Defepripron, Desipramin, Desirudin, Desloaratadin, Desmopressin, Desogestrel, Desonid, Dexibuprofen, Dexketoprofen, Disoproxil, Diazepam und Diazepamderivate, Dihydralazin, Diltiazem, Dimenhydrinat, Dimethylsulfoxid, Dimeticon, Dipivoxil, Dipyridarnoi, Dolasetron, Domperidon und Domperidanderivate, Donepzil, Dopamin, Doxazosin, Doxorubizin, Doxylamin, Diclofenac, Divalproex, Dronabinol, Drospirenon, Drotrecogin alpha, Dutasterid, Ebastin, Econazol, Efavirenz, Eletripan, Emidastin, Emtricitabin, Enalapril, Encepur, Entacapon, Enfurvirtid, Ephedrin, Epinephrin, Eplerenon, Epoetin und Epoetinderivate, Eprosartan, Eptifibatid, Ertapenem, Esomeprazol, Estrogen und Estrogenderivate, Etanercept, Ethenzamid, Ethinöstradiol, Etofenamat, Etofibrat, Etofyllin, Etonogestrel, Etoposid, Exemestan, Ezetimib, Famciclovir, Famotidin, Faropenandaloxat, Felodipin, Fenofibrat, Fentanyl, Fenticonazol, Fexofenadin, Finasterid, Fluconazol, Fludarabin, Flunarizin, Fluorouracil, Fluoxetin, Flurbiprofen, Flupirtin, Flutamid, Fluvastatin, Follitropin, Fomivirsen, Fondaparinux, Formoterol, Fosfomicin, Frovatriptan, Furosemid, Fusidinsäure, Gadobenat, Galantamin, Gallopamil, Ganciclovir, Ganirelix, Gatifloxacin, Gefitinib, Gemfibrozil, Gentamicin, Gepiron, Gestagen und Gestagenderivate, Ginkgo, Glatiramer, Glibenclamid, Glipizide, Glucagon, Glucitol und Glucitolclerivate, Glucosamin und Glucosaminderivate, Glykosidantibiotika, , Glutathion, Glycerol und Glycerolderivate, Hypothalamushormone, Goserelin, Grepafloxacin, Gyrasehemmer, Guanethidin, Gyrasehemmer, Hämin, Halofantrin, Haloperidol, Harnstoffderivate als orale Antidiabetika, Heparin und Heparinderivate, Herzglykoside, Hyaluronsäure, Hydralazin, Hydrochlorothiazid und Hydrochlorothiazidderivate, Hydroxyomeprazol, Hydroxyzin, Ibritumomab, lbuprofen, Idarubicin, Ifliximab, lfosfamid, Iloprost, Imatinib, Imidapril, Imiglucerase, Imipramin, lmiquimod, Imidapril, Indometacin, Indoramin, Infliximab, Insulin, Insulin glargin, Interferone, Irbesartan, Irinotecan, lsoconazol, Isoprenalin, Itraconazol, lvabradine, Jod und Jodderivate, Johanniskraut, Kaliumsalze, Ketoconazol, Ketoprofen, Ketotifen, Lacidipin, Lansoprazol, Laronidase, Latanoprost, Leflunomid, Lepirudin, Lercanidipin, Leteprinim, Letrozol, Levacetylmethadol, Levetiracetam, Levocetirizin, Levodopa, Levodrpropicin, Levomethadon, Licofelone, Linezolid, Lipinavir, Liponsäure und Liponsäurederivate, Lisinopril, Lisurid, Lofepramin, Lodoxamid, Lomefloxacin, Lomustin, Loperamid, Lopinavir, Loratadin, Lornoxicam, Losartan, Lumefantrin, Lutropine, Magnesiumsalze, Makrolidantibiotika, Mangafodipir, Maprotilin, Mebendazol, Mebeverin, Meclozin, Mefenaminsäure, Mefloquin, Meloxicam, Memantin, Mepindolol, Meprobamat,Meropenem, Mesalazin, Mesuximid, Metamizol, Metformin, Methadon, Methotrexat, Methyl-(5-amino-4-oxopentanoat), Methylnaloxon, Methylnaltrexone, Methylphenidat, Methylprednisolon, Metixen, Metoclopramid, Metoprolol, Metronidazol, Mianserin, Mibefradil, Miconazol, Mifepriston, Miglitol, Miglustad, Minocyclin, Minoxidil, Misoprostol, Mitomycin, Mizolastin, Modafinil, Moexipril, Montelukast, Moroctocog, Morphinane, Morphin und Morphinderivate, Moxifloxacin, Mutterkornalkaloide, Nalbuphin, Naloxon, Naproxen, Naratriptan, Narcotin, Natamycin, Nateglinid, Nebivolol, Nefazodon, Nelfinavir, Neostigmin, Neramexan, Nevirapin, Nicergolin, Nicethamid, Nifedipin, Nifluminsäure, Nimodipin, Nimorazol, Nimustin, Nesiritid, Nisoldipin, Norfloxacin, Novaminsulfon, Noscapin, Nystatin, Ofloxacin, Oktotride, Olanzapin, Olmesartan, Olsalazin, Oseltamivir, Omeprazol, Omoconazol, Ondansetron, Orlistat, Oseltamivir, Oxaceprol, Oxacillin, Oxaliplatin, Oxaprozin, Oxcarbacepin, Oxicodon, Oxiconazol, Oxymetazolin, Palivizumab, Palonosetron, Pantoprazol, Paracetamol, Parecoxib, Paroxetin, Pegaspargase, Peg-Interferon, Pegfilgrastrim, Penciclovir, orale Penicilline, Pentazocin, Pentifyllin, Pentoxifyllin, Peptidantibiotika, Perindopril, Perphenazin, Pethidin, Pflanzenextrakte, Phenazon, Pheniramin, Phenylbuttersäure, Phenytoin, Phenothiazine, Phenserin, Phenylbutazon, Phenytoin, Pimecrolimus, Pimozid, Pindolol, Pioglitazon, Piperazin, Piracetam, Pirenzepin, Piribedil, Pirlindol, Piroxicam, Pramipexol, Pramlintide, Pravastatin, Prazosin, Procain, Promazin, Propiverin, Propranolol, Propionsaurederivate, Propyphenazon, Prostaglandine, Protionamid, Proxyphyllin, Quetiapin, Quinapril, Quinaprilat, Quinupristin, Ramipril, Ranitidin, Rabeprazol, Raloxifen, Ranolazine, Rasburicase, Reboxetin, Repaclinide, Reproterol, Reserpin, Revofloxacin, Ribavirin, Rifampicin, Riluzole, Rimexolon, Risedronat, Risperidon, Ritonavir, Rituximab, Rivastimen, Risatriptan, Rofecoxib, Ropinirol, Ropivacain, Rosiglitazon, Roxatidin, Roxithromycin, Ruscogenin, Rosuvastatin, Rutosid und Rutosidderivate, Sabadilla, Salbutamol, Salicylate, Salmeterol, Saperconazole, Schilddrüsenhormone, Scopolamin, Selegilin, Sertaconazol, Sertindol, Sertralin, Sevelamer, Sibutramin, Sildenafil, Silikate, Simvastatin, Sirolimus, Sitosterin, Sotalol, Spagluminsäure, Sparfloxacin, Spectinomycin, Spiramycin, Spirapril, Spironolacton, Stavudin, Streptomycin, Sucralfat, Sufentanil, Sulbactam, Sulfonamide, Sulfasalazin, Sulpirid, Sultamicillin, Sultiam, Sumatriptan, Suxamethoniumchlorid, Tacrin, Tacrolimus, Tadalafil, Taliolol, Talsaclidin, Tamoxifen, Tasonermin, Tazaroten, Tegafur, Tegaserod, Telithromycin, Telmisartan, Temoporfin, Temozolomid, Tenatoprazol, Tenecteplase, Teniposid, Tenofovir, Tenoxicam, Teriparatid, Terazosin, Terbinafin, Terbutalin, Terfenadin, Teriparatid, Terlipressin, Tertatolol, Testosteron und Testosteronderivate, Tetracycline, Tetryzolin, Tezosentan, Theobromin, Theophyllin, Theophyllinderivate, Thiamazol, Thiotepa, Thr. Wachstumsfaktoren, Tiagabin, Tiaprid, Tibolon, Ticlopidin, Tilidin, Timolol, Tinidazol, Tioconazol, Tioguanin, Tiotropium, Tioxolon, Tirazetam, Tiropramid, Trofiban, Tizanidin, Tolazolin, Tolbutamid, Tolcapon, Tolnaftat, Tolperison, Tolterodin, Topiramat, Topotecan, Torasemid, Tramadol, Tramazolin, Trandolapril, Tranylcypromin, Trapidil, Trastuzumab, Travoprost, Trazodon, Trepostinil, Triamcinolon und Triamcinolonderivate, Triamteren, Trifluperidol, Trifluridin, Trimetazidine, Trimethoprim, Trimipramin, Tripelennamin, Triprolidin , Trifosfamid, Tromantadin, Trometamol, Tropalpin, Trovafloxacin, Troxerutin, Tulobuterol, Trypsine, Tyramin, Tyrothricin, Urapidil, Ursodeoxycholsäure, Theophyllin Ursodeoxycholsäure, Valaciclovir, Valdecoxib, Valganciclovir, Valproinsäure, Valsartan, Vancomycin, Vardenafil, Vecuroniumchlorid, Venlafaxin, Verapamil, Verteporfin, Vidarabin, Vigabatrin, Viloxazin, Vinblastin, Vincamin, Vincristin, Vindesin, Vinorelbin, Vinpocetin, Viquidil, Vitamin D und Derivate von Vitamin D, Voriconazol, Warfarin, Xantinolnicotinat, Ximelagatran, Xipamid, Zafirlukast, Zalcitabin, Zaleplon, Zanamivir, Zidovudin, Ziprasidon, Zoledronsäure, Zolmitriptan, Zolpidem, Zoplicon, Zotepin und dergleichen.

### Besonders bevorzugte Wirkstoffe

Beispiele besonders bevorzugter Wirkstoffe sind Metoprolol Succinat und Terbulatin Sulphat.

Die Wirkstoffe können gewünschtenfalls auch in Form ihrer pharmazeutisch annehmbaren Salze oder Derivate verwendet werden, und im Falle chiraler Wirkstoffe können sowohl optisch aktive Isomere als auch Racemate oder Diastereoisomerengemische eingesetzt werden. Gewünschtenfalls können die erfindungsgemässen Zusammensetzungen auch zwei oder mehrere pharmazeutische Wirkstoffe enthalten.

### Die äußere Kontrollschicht d)

Die äußere Kontrollschicht d), enthält zu mindestens 60, bevorzugt mindestens 80, besonders bevorzugt 90 bis 100 Gew.-% ein oder eine Mischung aus mehreren (Meth)acrylatcopolymeren, aus 98 bis 85 Gew.-% C₁- bis C₄-Alkylestern der (Meth)acrylsäure und 2 bis 15 Gew.-% Methacrylatmonomeren mit einer quaternären Ammoniumgruppe Im Alkylrest, und gegebenenfalls bis zu 40, bevorzugt bis 20, insbesondere 0 bis 10 Gew.-% weitere pharmazeutisch verwendbare Polymere. Besonders bevorzugt sind jedoch keine weiteren pharmazeutisch verwendbare Polymere enthalten. Die Angaben zu den Gew.-% der oben genannten Polymere in der äußeren Kontrollschicht d) berechnen sich dabei ohne Berücksichtigung eventuell zusätzlich enthaltener pharmazeutisch üblicher Hilfsstoffe.

Entsprechende (Meth)acrylat-Copolymere sind z. B. aus EP-A 181 515 oder aus DE-PS 1 617 751 bekannt. Es handelt sich um unabhängig vom pH-Wert lösliche oder quellbare Polymerisate, die für Arzneimittelüberzügen geeignet sind. Als mögliches Herstellungverfahren ist die Substanzpolymeriation in Gegenwart eines im Monomerengemisch gelösten radikalbildenden Initiators zu nennen. Ebenso kann das Polymerisat auch mittels Lösungs- oder Fällungspolymerisation hergestellt werden. Das Polymerisat kann auf diese Weise In Form eines feinen Pulvers erhalten werden, was bei der Subtanzpolymerisation durch Mahlen, bei Lösungs- und Fällungspolymerisation z. B. durch Sprühtrocknung erreichbar ist:

Das (Meth)acrylat-Copolymer, setzt sich aus 85 bis 98 Gew.-% radikalisch polymerisierten C₁- bis C₄-Alkylestern der Acryl- oder der Methacrylsäure und 15 bis 2 Gew.-% (Meth)acrylat-Monomeren mit einer quaternären Ammoniumgruppe im Alkylrest zusammen.

Bevorzugte C₁- bis C₄-Alkylestern der Acryl- oder der Methacrylsäure sind Methylacrylat, Ethylacrylat, Butylacrylat, Butylmethacrylat und Methylmethacrylat.

Als (Meth)acrylat Monomer mit quaternären Ammoniumgruppen wird 2-Trimethylammoniumethylmethacrylat-Chlorid besonders bevorzugt.

Ein entsprechendes Copolymer, kann z. B. aus 50 - 70 Gew.-% Methylmethacrylat, 20 - 40 Gew.-% Ethylacrylat und 7 - 2 Gew.-% 2-Trimethylammoniumethylmethacrylat-Chlorid aufgebaut sein.

Ein konkret geeignetes Copolymer enthält 65 Gew.-% Methylmethacrylat, 30 Gew.-% Ethylacrylat und 5 Gew.-% 2-Trimethylammoniumethylmethacrylat-Chlorid aufgebaut sein (EUDRAGIT® RS).

Ein weiteres geeignetes (Meth)acrylat-Copolymer kann z. B. aus 85 bis weniger als 93 Gew.-% C1- bis C4-Alkylestern der Acryl- oder der Methacrylsäure und mehr als 7 bis 15 Gew.-% (Meth)acrylat Monomeren mit einer quaternären Ammoniumgruppe im Alkylrest aufgebaut sein. Derartige (Meth)acrylatMonomere sind handelsüblich und werden seit langem für retardierende Überzüge verwendet.

Ein konkret geeignetes Copolymer enthält z. B. 60 Gew.-% Methylmethacrylat, 30 Gew.-% Ethylacrylat und 10 Gew.-% 2-Trimethylammoniumethlymethacrylat-Chlorid (EUDRAGIT® RL).

Gegebenenfalls können in der aüßeren Kontrollschicht d) bis zu 40, bevorzugt bis 20, insbesondere 0 bis 10 Gew.-% weitere pharmazeutisch verwendbare Polymere enthalten sein. Geeignete Polymere sind z. B.:

Copolymere aus Methylmethacrylat und/oder Ethylacrylat und Methacrylsäure, Copolymere aus Methylmethacrylat, Methylacrylat und Methacrylsäure, Copolymere aus Methylmethacrylat, Butylmethacrylat und Dimethylethylmethacrylat, Copolymere aus Methylmethacrylat, Ethylacrylat und Trimethylammoniumethylmethacrylat, Copolymere aus Methylmethacrylat und Ethylacrylat, Copolymere aus Ethylacrylat, Methylacrylat, Butylmethacrylat und Methacrylsäure

Polyvinylpyrolidone (PVP), Polyvinylalkohole, Polyvinylalkohol-Polyethylenglycol-Graft-Copolymer (Kollicoat®), Stärke und deren Derivate, Polyvinylacetatphtalat (PVAP, Coateric®), Polyvinylacetat (PVAc, Kollicoat), Vinylacetat-Vinylpyrolidon-Copolymer (Kollidon® VA64), Vinylacetat : Crotonsäure-Copolymer 9:1 (VAC : CRA, Kollicoat® VAC), Polyethylenglykole mit einem Molekulargewicht über 1000 (g/mol), Chitosan, ein (Meth)acrylatcopolymer, bestehend aus 20 - 40 Gew.-% Methylmethacrylat und 60 bis 80 Gew.-% Methacrylsäure, eine vernetzte und/oder unvernetzte Polyacrylsäure, ein Na-Alginat, und/oder ein Pektin,

Cellulosen wie z. B. anionische Carboxymethylcellulose und deren Salze (CMC, Na-CMC, Ca-CMC, Blanose, Tylopur), Carboxymethylethylcellulose (CMEC, Duodcell®), Hydroxyethylcellulose (HEC, Klucel), Hydroxypropylcellulose (HPC), Hydroxypropylmethylcellulose (HPMC, Pharmacoat, Methocel, Sepifilm, Viscontran, Opadry,), Hydroxymrthylethylcellulose (HEMC), Ethylcellulose (EC, Ethocel^{®}, Aquacoat^{®}, Surelease^{®}), Methylcellulose (MC, Viscontran, Tylopur, Methocel), Celluloseester, Celluloseglycolat, Celluloseacetatphtalat (CAP, Cellulosi acetas, PhEur, Celluloseacetate-phtalate, NF, Aquateric®), Celluloseacetatsuccinat (CAS), Celluloseacetattrimeliat (CAT), Hydroxypropylmethylcellulosephtalat (HPMCP, HP50, HP55), Hydroxypropylmethylcelluloseacetatsuccinat (HPMCAS -LF, -MF, -HF).

### Schichtdicken und Gewichtsanteile

### Kernschicht a)

Die Kemschicht a) (ohne Nonparielles) kann einen mittleren Durchmesser im Bereich von etwa 100 bis 800, bevorzugt 250 bis 500 µm (entsprechend einem Bereich von etwa 60 bis 40 Mesh) aufweisen.

### Innere Kontroll-Schicht b)

Die innere Kontroll-Schicht b) kann einen Gewichtsanteil von 0,5 bis 80, bevorzugt 2,5 bis 50, besonders bevorzugt 5 bis 40 Gew.-% bezogen auf die Kernschicht a) aufweisen. Die Schichtdicke beträgt günstigerweise etwa 1 bis 100, bevorzugt 5 bis 50, insbesondere 10 bis 40 µm.

### Wirkstoff-Schicht c)

Die Wirkstoff-Schicht c) kann 10 bis 400, bevorzugt 50 bis 200 Gew.-% bezogen auf die Kernschicht a) und die innere Kontroll-Schicht b) ausmachen.

### Äußere Kontroll-Schicht d)

Die äußere Kontroll-Schicht d) kann einen Gewichtsanteil von 2,5 bis 100, bevorzugt 10 bis 70, besonders bevorzugt 20 bis 60 Gew.-% bezogen auf die Kernschicht a), die innere Kontroll-Schicht b) und die Wirkstoffschicht c) aufweisen. Die Schichtdicke beträgt etwa 4 bis 150, insbesondere 15 bis 75, besonders bevorzugt 30 bis 70 µm.

### Pharmazeutisch übliche Hilfsstoffe

Die Schichten a), b), c) und d) können zusätzlich und in an sich bekannter Weise pharmazeutisch übliche Hilfsstoffe enthalten..

Der erfindungsgemäßen Formulierung werden bevorzugt bei der Herstellung der Granulate oder Pulver pharmazeutisch übliche Hilfsstoffe, gelegentlich auch als übliche Zuschlagstoffe bezeichnet, hinzugefügt. Grundsätzlich müssen natürlich alle eingesetzten Substanzen toxikologisch unbedenklich und insbesondere in Arzneimitteln ohne Risiko für Patienten zu verwenden sein.

Einsatzmengen und Verwendung der pharmazeutisch üblichen Hilfsstoffe in Arzneimittelüberzügen oder Beschichtungen sind dem Fachmann geläufig. Pharmazeutisch übliche Hilfsstoffe oder Zuschlagstoffe können z. B. Trennmittel, Pigmente, Stabilisatoren, Antioxidantien, Porenbildner, Penetrationsförderer, Glanzmittel, Aromastoffe oder Geschmacksmittel sein. Sie dienen als Verarbeitungshilfsmittel und sollen ein sicheres und reproduzierbares Herstellungsverfahren sowie gute Langzeitlagerstabilität gewährleisten oder sie erreichen in der Arzneiform zusätzliche vorteilhafte Eigenschaften. Sie werden den Polymerzubereitungen vor der Verarbeitung zugesetzt und können die Permeabilität der Überzüge beeinflussen, was ggf. als zusätzlicher Steuerparameter genutzt werden kann.

### Trennmittel:

Trennmittel besitzen in der Regel lipophile Eigenschaften und werden in der Regel den Sprühsuspensionen zugesetzt. Sie verhindern eine Agglomeration der Kerne während der Befilmung. Bevorzugt werden Talkum, Mg- oder Ca - Stearat, gemahlene Kieselsäure, Kaolin oder nicht ionische Emulgatoren mit einem HLB - Wert zwischen 3 und 8 eingesetzt. Übliche Einsatzmengen für Trennmittel liegen zwischen 0,5 bis 100 Gew.-% bezogen auf das Kerngewicht.

### Pigmente:

Mit dem Überzugsmittel unverträgliche Pigmente sind insbesondere solche Pigmente, die wenn sie der (Meth)acrylat-Copolymer-Dispersion direkt zugesetzt werden, z. B. durch Einrühren, in üblichen Anwendungsmengen von z. B. 20 bis 400 Gew.-% bezogen auf das Trockengewicht des (Meth)acrylat-Copolymeren zur Destabilisierung der Dispersion, Koagulation, zu Entmischungserscheinungen oder ähnlich unerwünschten Effekten führen. Weiterhin sind die zu verwendenden Pigmente natürlich nicht toxisch und für pharmazeutische Zwecke geeignet. Siehe dazu z. B. auch: Deutsche Forschungsgemeinschaft, Farbstoffe für Lebensmittel, Harald Boldt Verlag KG, Boppard (1978); Deutsche Lebensmittelrundschau 74, Nr. 4, S. 156 (1978); Arzneimittelfarbstoffverordnung AmFarbV vom 25.08.1980.

Mit dem Überzugsmittel unverträgliche Pigmente können z. B. Aluminiumoxidpigmente sein. Unverträgliche Pigmente sind z. B., Gelborange, Cochenillerotlack, Farbpigmente auf Basis von Aluminiumoxid bzw Azofarbstoffen, Sulfonsäurefarbstoffe, Gelborange S (E110, C.I. 15985, FD&C Yellow 6), Indigocarmin (E132, C.I. 73015, FD&C Blue 2), Tartrazin (E 102, C.I. 19140, FD&C Yellow 5), Ponceau 4R (E 125, C.I. 16255, FD&C Cochineal Red A), Chinolingleb (E 104, C.I. 47005, FD&C Yellow 10), Erythrosin (E127, C.I. 45430, FD&C Red 3), Azorubin (E 122, C.I. 14720, FD&C Carmoisine), Amaranth (E 123, C.I. 16185, FD&C Red 2), Brilliantsäuregrün (E 142, C.I. 44090, FD&C Green S).

Die angegebenen E-Nummern der Pigmente beziehen sich auf eine EU-Nummerierung. Siehe dazu auch "Deutsche Forschungsgemeinschaft, Farbstoffe für Lebensmittel, Harald Boldt Verlag KG, Boppard (1978); Deutsche Lebensmittelrundschau 74, Nr. 4, S. 156 (1978); Arzneimittelfarbstoffverordnung AmFarbV vom 25.08.1980. Die FD&C-Nummern beziehen sich auf die Zulassung in Food, Drugs und Cosmetics durch U.S. Food and Drug Administration (FDA) beschrieben in: U.S. Food and Drug Administration, Center for Food Safety and Applied Nutrition, Office of Cosmetics and Colors: Code of Federal Regulations - Title 21 Color Additive Regulations Part 82, Listing of Certified Provisionally Listed Colors and Specifications (CFR 21 Part 82).

### Weichmacher

Weitere Zuschlagstoffe können auch Weichmacher sein. Übliche Mengen liegen zwischen 0 und 50, bevorzugt 5 bis 20 Gew.-% bezogen z. B. auf das (Meth)acrylatcopolymer der äußeren Schicht d).

Weichmacher können je nach Typ (lipophil oder hydrophil) und zugesetzter Menge die Funktionalität der Polymerschicht beeinflussen. Weichmacher erreichen durch physikalische Wechselwirkung mit dem Polymeren eine Absenkung der Glasübergangstemperatur und fördern in Abhängigkeit von der zugesetzten Menge die Verfilmung. Geeignete Stoffe haben in der Regel ein Molekulargewicht zwischen 100 und 20.000 und enthalten eine oder mehrere hydrophile Gruppen im Molekül, z. B. Hydroxyl-, Ester- oder Aminogruppen. Beispiele geeigneter Weichmacher sind Citronensäurealkylester, Glycerinester, Phthalsäurealkylester, Sebacinsäurealkylester, Succroseester, Sorbitanester, Diethylsebacat, Dibutylsebacat und Polyethylenglykole 200 bis 12.000. Bevorzugte Weichmacher sind Triethylcitrat (TEC), Acetyltriethylcitrat (ATEC) und Dibutylsebacat (DBS). Weiterhin zu nennen sind in der Regel bei Raumtemperatur flüssige Ester wie Citrate, Phthalate, Sebacate oder Rizinusöl. Bevorzugt werden Zitronensäure- und Sebacinsäureester verwendet.

Die Zugabe der Weichmacher zur Formulierung kann in bekannter Weise, direkt, in wäßriger Lösung oder nach thermische Vorbehandlung der Mischung vorgenommen werden. Auch können Mischungen von Weichmachern eingesetzt werden.

### Verfahren zur Herstellung einer mehrschichtigen Arzneiform

Die mehrschichtige Arzneiform kann in an sich bekannter Weise mittels üblicher pharmazeutischer Verfahren wie direktes Verpressen, Verpressen von Trocken-, Feucht- oder Sintergranulaten, Extrusion und anschließende Ausrundung, feuchte oder trockene Granulation oder direkte Pelletierung (z.B. auf Tellern) oder durch Binden von Pulvern (Powder layering) auf wirkstofffreie Kugeln bzw. Kerne (Nonpareilles) oder wirkstoffhaltige Partikeln, mittels Sprühverfahren oder Wirbelschichtgranulation hergestellt werden. Das Auftragen der inneren und äußeren Kontrollschichten b) und d) kann nach bekannten und üblichen Verfahren, wie z. B. Sprühauftrag von Polymerlösungen oder Polymerdispersionen erfolgen.

### Beispielhafte Standardprozessparameter

Die nachfolgenden Standardprozessparameter sollen mögliche Vorgehensweisen beim Herstellungsverfahren beispielhaft erläutern.

### Stufe 1: (Formulierung einer Kernschicht a))

Für die Versuche werden Kristall-Kerne ausgewählt, die im Bereich von 400 µm -800 µm lagen.

### Stufe 2: (Auftrag einer Inneren Kontrollschicht b))

Modulatorschicht mit EUDRAGIT® NE (Copolymer aus 50 Gew.-% Methylmethacrylat und 50 Gew.-% Ethylacrylat)

20% w/w EUDRAGIT® NE 30 D Suspension wird als die Grundmodulierende Schicht für die meisten Versuche verwendet. Die Formulierung beinhaltet 15% Feststoff in Dispersion mit 20% Polymer, 5% Glycerolmonostearat (GMS-900), 2% Tween 80 und 0,5% eines Pigments.

Diese Schicht wird mittels eines Wirbelschichtgeräts auf die Kristall-Kerne aufgebracht.

### Prozessparameter:

| | |
|---|---|
| Zuluftemperatur. | 32° C |
| Produkttemperatur. : | 30° C |
| Ablufttemperatur. : | 23° C |
| Pumpe-U/min: | 8-10 (5-10 g/min) |
| Prozesszeit: | 120-160 min. |
| Trocknungsverfahren: | 2 Std. in Umlufttrockenschrank bei 40° C |

### Stufe 3 (Auftrag einer Wirkstoffschicht c))

Der Wirkstoff kann auf einfachen Kristall-Kernen oder auf mit einer modulatorisch wirkenden Substanz überzogenen Kristall-Kernen aufgetragen werden, bis man eine Gewichtszunahme von 100 bis 200% erhält.
Der Wirkstoffauftrag kann auch mit zusätzlicher Salzintegration vorgenommen werden, um die Salzkonzentration in den Pellets zu steigern.
Der Wirkstoffauftrag wird z. B. im Dragierkessel mit dem bekannten "Powder-Layering" Verfahren durchgeführt.

### Allgemeine Prozessparametern für den Wirkstoffauftrag

| | |
|---|---|
| Sprühzeit | 90 min. |
| Gesamtvolumen | 543 g |
| Gewicht/Pulver in Portionen | 15 gm |
| Düse | 1,00 mm |
| Sprühdruck | Niedrig |
| Dragierkesselgeschwindigkeit | 24 -25 U/min |
| Pumpgeschwindigkeit | 12 U/min (9 g/min) |
| Trocknung im Gerät | 5 min. |
| Endtrocknung in Umlufttrockenschrank | 12 h bei 40°C |
| Abluftbedingungen | An |

Die so erhaltenen, mit Wirkstoff überzogenen Pellets können im Größenbereich von 600 - 1200 µm liegen und für eine weitere Beschichtung mit EUDRAGIT® RS (Copolymer aus 65 Gew.-% Methylmethacrylat, 30 Gew.-% Ethylacrylat und 5 Gew.-% 2-Trimethylammoniumethylmethacrylat-Chlorid) verwendet werden.

### Stufe 4 (Auftrag einer äußeren Kontrollschicht d), bestehend aus einem Retard Überzüge mit (EUDRAGIT® RS)

Die mit Wirkstoff überzogenen Pellets können beispielsweise mit EUDRAGIT® RS mit unterschiedlichen Auftragsmengen (von 10-50%) in Wirbelschichtgerät beschichtet werden. Eine Formulierung kann z. B. beinhalten: 20% Feststoff in EUDRAGIT^{®} RS-Dispersion mit 50% Talkum, 20% Triethylcitrat, 0,5% Pigmente.

### Prozessparameter

| | |
|---|---|
| Zuluftemperatur. | 35° C |
| Produkttemperatur. : | 32° C |
| Ablufttemperatur. : | 24° C |
| Pumpe-U/min: | 8-16 (4-8 g/min) |
| Prozesszeit: | 120-180 min. |
| Trocknungsverfahren: | 2 Std. in Umlufttrockenschrank bei 40° C |

Spezifische Beispiele:

### Beispiel I

### Modulierte Schichtkonzentration bis 10%ig w/w:

Trinatriumcitratkristalle wurden mit 10% W/W EUDRAGIT® NE 30D beschichtet. Auf diese Schicht wird Theophyllin bis 200 % Gewichtszunahme aufgebracht. Diese beschichteten Kerne werden weiter überzogen mit 20-40% w/w EUDRAGIT® RS30D

### Beispiel II

### Modulierte Schichtkonzentration bis 20%ig W/W:

Trinatriumcitratkristalle werden mit 20% W/W EUDRAGIT® NE 30D beschichtet. Auf diese Schicht wird Theophyllin bis 200 % Gewichtszunahme aufgebracht. Diese beschichteten Kerne werden weiter überzogen mit 20-40% w/w EUDRAGIT® RS30D

### Beispiel III

### Steigerung des Salzeskonzentration im fertigen Pellet:

Natriumchloridkerne wurden zuerst mit einer Modulatorschicht von EUDRAGIT® NE 30 D bis zu 20% w/w beschichtet. Auf diese Schicht wurde Theophyllin und gemahlenen Natriumchlorid Kristallen bis zu 200% Gewichtszunahme aufgebracht. Diese beschichteten Pellets wurden weiter überzogen mit 20-40% w/w EUDRAGIT® RS30D.

### Beispiel IV

### Wirkung von verschiedenen Salzen:

Natriumchlorid- und Natriumacetatkristalle werden zuerst mit einer EUDRAGIT® NE 30 D bis zu 20% w/w beschichtet. Auf diese Schicht wird Theophyllin bis zu 200% Gewichtszunahme aufgebracht. Diese beschichteten Pellets werden weiter überzogen mit 20-40% w/w EUDRAGIT® RS30D.

### Mögliche Freisetzungscharakteristiken

Die mehrschichtige Arzneiform eignet sich insbesondere, um spezielle Wirkstoff-Freisetzungscharakteristiken zu realisieren. Zu nennen sind Wirkstoff-Freisetzungscharakteristiken nullter Ordnung (linear), 1. Ordnung (beschleunigt), schnell-langsam-, langsam-schnell-Freisetzungscharakteristiken.

### Arzneiform für den Wirkstoff Metoprolol-Succinat

Beim Wirkstoff Metoprolol-Succinat, der zur Therapie von Hypertension und Angina eingesetzt werden kann, ist die Formulierung einer Arzneiform von Vorteil, die vor dem zu Bett gehen eingenommen, den Wirkstoff zunächst linear freisetzt, aber nach 4 bis 6 Stunden zu einer beschleunigten Wirkstoffabgabe übergeht. Damit lässt sich der Gefahr von hohem Blutdruck und Herzinfarkten, die in den frühen Morgenstunden besonders hoch ist, begegnen.

Es werden erfindungsgemäß vier mögliche Varianten offenbart, die mit denen sich die gewünschte Freisetzungscharakteristik für den Wirkstoff Metoprolol Succinat erreichen lässt.

| | ***Beispiel M1*** | ***Beispiel M2*** | ***Beispiel M3*** | ***Beispiel M4*** |
|---|---|---|---|---|
| Kernschicht a) | Na-Acetat Kristalle | NaCl-Kristalle | NaCl-Kristalle | NaCl-Kristalle |
| Innere Kontrollschicht b) | 20 Gew.-% EUDRAGIT® NE | 20 Gew.-% EUDRAGIT® NE | 40 Gew.-% EUDRAGIT® NE | 20 Gew.-% EUDRAGIT® NE |
| [Gew.-% bez. auf a)] | | | | |
| Wirkstoffschicht c) | 200 Gew.-% Metoprolol-Succinat | 200 Gew.-% Metoprolol-Succinat | 200 Gew.-% Metoprolol-Succinat | 200 Gew.-% Metoprolol-Succinat + NaCl |
| [Gew.-% bez. auf a) + b)) | | | | |
| Äußere Kontrollschicht d) | 40 Gew.-% EUDRAGIT^{®} RS | 50 Gew.-% EUDRAGIT^{®} RS | 50 Gew.-% EUDRAGIT^{®} RS | 50 Gew.-% EUDRAGIT^{®} RS |
| [Gew.-% bez. auf a), b) + c)] | | | | |

| | | | | |
|---|---|---|---|---|
| EUDRAGIT® RS = Copolymer aus 65 Gew.-% Methylmethacrylat, 30 Gew.-% Ethylacrylat und 5 Gew.-% 2-Trimethylanimoniumethytmethacrylat-Chlorid. EUDRAGIT® NE = Copolymer aus 50 Gew.-% Methylmethacrylat und 50 Gew.-% Ethylacrylat. | | | | |

Die Freisetzungscharakteristik der Pellets aus Beispiel M4 wurde im Test nach USP <711> Dissolution, Apparatus 1, Phosphatpuffer pH 6,8 geprüft. Dabei wurde festgestellt, dass bis zur zweiten und von der zweiten bis zur vierten Stunde jeweils ca. 11 % des enthaltenen Wirkstoffs freigesetzt wurde. Ab der vierten Stunde war eine beschleunigte Wirkstoffabgabe von ca. 15 % bis zur sechsten Stunde und jeweils 20 % von der sechsten zur achten und der achten zur zehnten Stunde zu beobachten. Ab der zehnten Stunde verlangsamte sich die Wirkstoffabgabe wieder.

| Metoprolol-Succinat Freistezung der Pellets aus Beispiel M4 | | |
|---|---|---|
| (USP I, 100 Upm, ph 6,8) | | |
| Stunde | Wirkstoffabgabe im 2 Stunden-Intervall | Wirkstoffabgabe kummuliert |
| 2 | 11 | 11 |
| 4 | 11 | 22 |
| 6 | 15 | 37 |
| 8 | 20 | 57 |
| 10 | 20 | 77 |
| 12 | 11 | 88 |

### Arzneiform für den Wirkstoff Terbutalin-Sulfat

Der Wirkstoff Terbutalin-Sulfat ist ein Beta 2 Antagonist, der zur Therapie von Astma eingesetzt werden kann. Erfindungsgemäß wird eine Formulierung mit annähernd konstanter Wirkstoffabgabegeschwindigkeit bereitgestellt. Dadurch können werden akute Astmasymtome bereits unmittelbar nach Einnahme der Arzneiform gemildert. Danach werden gleichmäßige Mengen des Wirkstoffs zur Unterdrückung des Wiederhochkommens weiterer Symtome abgegeben. Es ist daher nicht notwendig mehrmals täglich, immer wieder sowie mehr oder weniger pünktlich Einzeldosen zu verabreichen, wie dies bei den meisten Arzneiformen des Stand der Technik der Fall ist. Dies ist für den Patienten insgesamt bequemer, akzepzabler (Patient Compliancy) und in vielen Fällen auch verträglicher.
Es werden erfindungsgemäß zwei mögliche Varianten offenbart, die mit denen sich die gewünschte Freisetzungscharakteristik für den Wirkstoff Terbutalin-Sulfat erreichen lässt.

| | ***Beispiel T1*** | ***Beispiel T2*** |
|---|---|---|
| Kernschicht a) | Na-Acetat Kristalle | NaCl-Kristalle |
| Innere Kontrollschicht b) | 20 Gew.-% EUDRAGIT^{®} NE | 20 Gew.-% EUDRAGIT^{®} NE |
| [Gew.-% bez. auf a)] | | |
| Wirkstoffschicht c) | 200 Gew.-% Terbutalin-Sulfat | 200 Gew.-% Terbutalin-Sulfat + NaCl |
| [Gew.-% bez. auf a) + b)] | | |
| Äußere Kontrollschicht d) | 30 Gew.-% EUDRAGIT^{®} RS | 30 Gew.-% EUDRAGIT^{®} RS |
| [Gew.-% bez. auf a), b) + c)] | | |

| | | |
|---|---|---|
| EUDRAGIT® RS = Copolymer aus 65 Gew.-% Methylmethacrylat. 30 Gew.-% Ethylacrylat und 5 Gew.-% 2-Trimethylammoniumethylmethacrylat-Chlorid. EUDRAGIT® NE = Copolymer aus 50 Gew.-% Methylmethacrylat und 50 Gew.-% Ethylacrylat. | | |

Die Freisetzungscharakteristik der Pellets aus Beispiel M4 wurde im Test nach USP <711 > Dissolution, Apparatus 1, Phosphatpuffer pH 6,8 geprüft. Dabei wurde festgestellt, dass in 2 Stunden-Intervallen in etwa konstante Wirkstoffmengen freigesetzt werden.

| Terbutalin-Sulfat Freisetzung der Pellets aus Beispiel T2 | | |
|---|---|---|
| (USP I, 100 Upm, ph 6,8). | | |
| Stunde | Wirkstoffabgabe im 2 Stunden-Intervall | Wirkstoffabgabe in % kummuliert |
| 2 | 14 | 14 |
| 4 | 17 | 31 |
| 6 | 14 | 45 |
| 8 | 10 | 55 |
| 10 | 9 | 64 |
| 12 | 10 | 74 |

### Darreichungsformen/Verwendungen

Die erfindungsgemäßen mehrschichtigen Arzneiformen liegen zunächst in Form von Tabletten oder Pellets vor. Diese können wiederum als Bestandteil einer multipartikulären Arzneiform, von pellethaltigen Tabletten, Minitabletten, Kapseln, Sachets, Brausetabletten oder Trockensäften verwendet werden. Erfindungsgemäß multipartikuläre Arzneiformen können insbesondere auch Mischungen von formulierten Pellets beinhalten, die verschiedene Wirkstoffe enthalten. Weiterhin können erfindungsgemäße multipartikuläre Arzneiformen mit ein und demselben Wirkstoff beladene Pelletpopulationen enthalten, die unterschiedlich formuliert sind und unterschiedliche Freisetzungsprofile aufweisen. Auf diese Weise kann man gemischte Freisetzungsprofile von einem oder mehreren Wirkstoffen erreichen und über die Mischungen eine nochmals feinere Anpassung für die gewünschte Therapie vornehmen.

### BEISPIELE

EUDRAGIT® RS = Copolymer aus 65 Gew.-% Methylmethacrylat, 30 Gew.-% Ethylacrylat und 5 Gew.-% 2-Trimethylammoniumethylmethacrylat-Chlorid.
EUDRAGIT® NE = Copolymer aus 50 Gew.-% Methylmethacrylat und 50 Gew.-% Ethylacrylat.

### Beispiele 1 - 5 (nicht erfindungsgemäß)

Um den Einfluß verschiedener modulatorisch wirkender Substanzen auf die äußere Kontrollschicht d) zu prüfen, wurden Pellets ohne eine innere Kontrollschicht b) hergestellt. Als Vergleich dienen Pellets ohne modulatorisch wirkender Substanzen mit mikrokristalliner Cellulose (Beispiel 5). Auf diese Weise können Effekte wie eine beschleunigte oder eine verlangsamte Wirkstoffabgabe unabhängig von einer inneren Kontrollschicht festgestellt werden.

Auf 700 g Kernmaterial werden in einem Dragierkessel eine Mischung aus 1290 g Theophyllin Pulver, 65 g Kollidon 25 und 6,5 g Aerosil 200 aufgestreut und durch gleichzeitiges Sprühen einer Lösung aus 33 g Theophyllin und 10 Kollidon 25 in 500 g demineralisiertem Wasser an das Kernmaterial gebunden. Auf 600 g der so hergestellten Theophyllin Pellets mit nicht retardiertem Modulator Kern wird in einer Wirbelschichtanlage eine Sprühsuspension aus 400 g EUDRAGIT RS 30 D (entsprechend 120 g Polymer), 60 g Talkum, 24 g Triethylcitrat, 0,6 g Eisenoxid Gelb und 538,3 g demineralisiertem Wasser aufgetragen. Die aufgetragene Polymermenge entspricht somit 20 % des Ausgangsmaterials.

Die unter Beispiel 1 - 5 hergestellten Pellets wurden in einem USP Dissolutionstester in einem PhEur Phosphatpuffer pH 6.8 hinsichtlich Wirkstoffabgabe untersucht:

| Beispiel | 1 | 2 | 3 | 4 | 5 |
|---|---|---|---|---|---|
| Kernschicht a) | Natrium-Acetat Kristalle | Natrium-Chlorid Kristalle | Natrium-Succinat Kristalle | Zitronen-Säure Kristalle | Mikro-Kristalline Cellulose Granulat |
| Innere Kontroll-Schicht b) | - | - | -- | - | - |
| Wirkstoff-Schicht c) | Theophyllin | Theophyllin | Theophyllin | Theophyllin | Theophyllin |
| Äußere Kontroll-Schicht d) | EUDRAGIT RS 30 D | EUDRAGIT RS 30 D | EUDRAGIT RS 30 D | EUDRAGIT RS 30 D | EUDRAGIT RS 30 D |
| Zeit [Std.] | | | | | |
| 0 | 0 | 0 | 0 | 0 | 0 |
| 0,5 | 3,1 | 0,4 | 7,0 | 6,3 | 1,8 |
| 1 | 5,4 | 1,1 | 13,2 | 10,2 | 3,0 |
| 2 | 9,2 | 2,1 | 28,2 | 18,1 | 5,2 |
| 4 | 14,8 | 3,9 | 65.9 | 35,1 | 11,6 |
| 6 | 20,1 | 5,5 | 77,9 | 51,0 | 20,7 |
| 8 | 25,0 | 7,1 | 89,7 | 66,8 | 30,9 |
| 10 | 29,1 | 8,4 | 96,3 | 80,0 | 42,7 |

Die Freigabewerte zeigen den für Diffusionsprozesse charakteristischen Verlauf 1. Ordnung. Ohne eine Kontrolle der Modulatorfreisetzung ergibt sich also sehr schnell ein Gleichgewicht im Überzogenen Pellet, welches die Permeabilität des Endüberzugs zu Beginn der Freigabe endgültig einstellt.

Das Freigabeprofil der Pellets mit mikrokristalliner Cellulose (Bsp. 5) liegt zwischen denen mit Natriumacetat und Natriumchlorid. Damit ergibt sich für Natriumacetat, Zitronensäure und Natriumsuccinat eine beschleunigende Wirkung und für Natriumchlorid eine reduzierende Wirkung.

### Beispiele 6-10

### (Erfindungsgemäß, "Linear" Freisetzungscharakteristik nullter Ordnung).

1000 g Kernmaterial werden in einer Wirbelschichtanlage mit einer Sprühsuspension aus 666 g EUDRAGIT NE 30 D (entsprechend 200 g Polymer), 4 g Polysorbat 80, 10 g Glycerolmonostearat, 1g Eisenoxid Gelb und 720 g demineralisiertem Wasser überzogen. Die aufgetragene Polymermenge entspricht somit 20 % des Ausgangsmaterials.
Auf 700 g der so hergestellten Kerne mit retardierter Modulatorabgabe werden in einem Dragierkessel eine Mischung aus 1290 g Theophyllin Pulver, 65 g Kollidon 25 und 6,5 g Aerosil 200 aufgestreut und durch gleichzeitiges Sprühen einer Lösung aus 33 g Theophyllin und 10 Kollidon 25 in 500 g demineralisiertem Wasser an das Kernmaterial gebunden.
Auf 600 g der so hergestellten Theophyllin Pellets mit retardiertem Modulator Kern wird in einer Wirbelschichtanlage eine Sprühsuspension aus 400 g EUDRAGIT® RS 30 D (entsprechend 120 g Polymer), 60 g Talkum, 24 g Triethylcitrat, 0,6 g Eisenoxid Gelb und 538,3 g demineralisiertem Wasser aufgetragen. Die aufgetragene Polymermenge entsprach somit 20 % des Ausgangsmaterials.

Die unter Beispiel 6 - 10 hergestellten Pellets wurden in einem USP Dissolutionstester in einem PhEur Phosphatpuffer pH 6.8 hinsichtlich Wirkstoffabgabe untersucht.:

| **Beispiel** | **6** | **7** | **8** | **9** | **10** |
|---|---|---|---|---|---|
| Kemschicht a) | Natrium-Acetat Kristalle | Natrium-Chlorid Kristalle | Natrium-Citrat Kristalle | Natrium-Succinat Kristalle | Zitronen-Säure Kristalle |
| Innere Kontroll-Schicht b) | EUDRAGIT NE 30 D | EUDRAGIT NE 30 D | EUDRAGIT NE 30 D | EUDRAGIT NE 30 D | EUDRAGIT NE 30 D |
| Wirkstoff-Schicht c) | Theophyllin | Theo-phyllin | Theo-phyllin | Theo-phyllin | Theo-phyllin |
| Äußere Kontroll-Schicht d) | EUDRAGIT RS 30 D | EUDRAGIT RS 30 D | EUDRAGIT RS 30 D | EUDRAGIT RS 30 D | EUDRAGIT RS 30 D |
| **Zeit [Std.]** | | | | | |
| **Wirkstoffabgabe [%]** | | | | | |
| 0 | 0 | 0 | 0 | 0 | 0 |
| 0,5 | 1,7 | 3,2 | 6,7 | 11,6 | 29,3 |
| 1 | 3,1 | 6,3 | 16,4 | 21,9 | 57,7 |
| 2 | 6,4 | 14,5 | 39,2 | 75,9 | 87,9 |
| 4 | 16,1 | 27,5 | 75,4 | 99,0 | 94,3 |
| 6 | 23,2 | 40,0 | 90,4 | | |
| 8 | 29,9 | 48,6 | | | |
| 10 | 38,2 | 63,6 | | | |

Die Freigabewerte zeigen einen Verlauf nullter Ordnung d.h. sie sind nahezu linear. Die Modulatorfreisetzung aus der Kernschicht a) verhindert somit im Falle von Natriumsuccinat und Zitronensäure die frühe Wirkstoffabgabe aus dem System, wodurch die beschleunigende Wirkung über einen längeren Zeitraum erhalten bleibt. Im Falle von Natriumcitrat und Natriumacetat wird die höchst mögliche Permeabilitätserhöhung des EUDRAGIT® RS Überzuges durch Verzögerung der Modulatoranlieferung nie erreicht, durch konstante Nachlieferung ergibt sich daher eine längere und lineare Freigabekurve verglichen mit dem unkontrollierten Modulator aus Beispiel 1 und 3.
Im Falle des Natriumchloridkerns bleibt reduzierende Wirkung durch eine konstante Nachlieferung länger erhalten, wodurch eine langsamere lineare Freigabe erzielt wird.

### Beispiel 11 (nicht erfindungsgemäß)

Zur Prüfung der These, das die gefundenen Steuerungsmöglichkeiten den Einsatz eines ionischen Überzugsmaterials erfordern wurden in den folgenden Beispielen Pellets mit einem neutralen Überzugsmaterial untersucht.

Auf 700 g Natriumacetat Kristalle werden in einem Dragierkessel eine Mischung aus 1290 g Theophyllin Pulver, 65 g Kollidon 25 und 6,5 g Aerosil 200 aufgestreut und durch gleichzeitiges Sprühen einer Lösung aus 33 g Theophyllin und 10 Kollidon 25 in 500 g demineralisiertem Wasser an das Kernmaterial gebunden.
Auf 600 g der so hergestellten Theophyllin Pellets mit nicht retardiertem Modulator Kern wurde in einer Wirbelschichtanlage eine Sprühsuspension aus 400 g EUDRAGIT^{®} NE 30 D (entsprechend 120 g Polymer), 2,4 g Polysorbat 80, 6 g Glycerolmonostearat, 0,6 g Eisenoxid Gelb und 432 g demineralisiertem Wasser aufgetragen.

### Beispiel 12 (nicht erfindungsgemäß)

Auf 700 g Natriumchlorid Kristalle werden in einem Dragierkessel eine Mischung aus 1290 g Theophyllin Pulver, 65 g Kollidon 25 und 6,5 g Aerosil 200 aufgestreut und durch gleichzeitiges Sprühen einer Lösung aus 33 g Theophyllin und 10 Kollidon 25 in 500 g demineralisiertem Wasser an das Kernmaterial gebunden.
Auf 600 g der so hergestellten Theophyllin Pellets mit nicht retardiertem Modulator Kern wurde in einer Wirbelschichtanlage eine Sprühsuspension aus 400 g EUDRAGIT^{®} NE 30 D (entsprechend 120 g Polymer), 2,4 g Polysorbat 80, 6 g Glycerolmonostearat, 0,6 g Eisenoxid Gelb und 432 g demineralisiertem Wasser aufgetragen.

| **Beispiel** | **1** | **6** | **11** | **12** |
|---|---|---|---|---|
| Kernschicht a) | Natrium-Acetat Kristalle | Natrium-Acetat Kristalle | Natrium-Acetat Kristalle | Natrium-Acetat Kristalle |
| Innere Kontroll-Schicht b) | - | EUDRAGIT® NE 30 D | - | EUDRAGIT® NE 30 D |
| Wirkstoff-Schicht c) | Theophyllin | Theophyllin | Theophyllin | Theophyllin |
| Äußere Kontroll-Schicht d) | EUDRAGIT® RS 30 D | EUDRAGIT® RS 30 D | EUDRAGIT® NE 30 D | EUDRAGIT® NE 30 D |
| | | | | |
| **Zeit [Std.]** | **Wirkstoffabgabe [%]** | | | |
| 0 | 0 | 0 | 0 | 0 |
| 0,5 | 3,1 | 1,7 | 8.96 | 6.74 |
| 1 | 5,4 | 3,1 | 14.66 | 11.56 |
| 2 | 9,2 | 6.4 | 22.61 | 18.67 |
| 4 | 14,8 | 16,1 | 38.33 | 32.11 |
| 6 | 20,1 | 23,2 | 58.51 | 48.90 |
| 8 | 25,0 | 29,9 | 73.78 | 66.01 |
| 10 | 29,1 | 38,2 | 82.35 | 75.74 |

■ Im Vergleich von Beispiel 1 mit 6 zeigt sich der Effekt der inneren Kontrollschicht b).
■ Im Vergleich von Beispiel 1 mit 11 zeigt sich der Effekt der erfindungsgemäßen äußeren Kontrollschicht d) in Beispiel 1.
■ Im Vergleich von Beispiel 11 mit 12 zeigt sich der Effekt des Fehlens einer erfindungsgemäßen äußeren Kontrollschicht d) unabhängig vom Vorhandensein einer inneren Kontrollschicht b).

### Beispiel 13 (Beschleunigt)

1000 g Natriumacetat Kristalle werden in einer Wirbelschichtanlage mit einer Sprühsuspension aus 666 g EUDRAGIT^{®} NE 30 D (entsprechend 200 g Polymer), 4 g Polysorbat 80, 10 g Glycerolmonostearat, 1g Eisenoxid Gelb und 720 g demineralisiertem Wasser überzogen. Die aufgetragene Polymermenge entsprach somit 20 % des Ausgangsmaterials.
Auf 700 g der so hergestellten Kerne mit retardierter Modulatorabgabe wurden in einem Dragierkessel eine Mischung aus 760 g Theophyllin Pulver, 560 g Natriumchlorid, 65 g Kollidon 25 und 6,5 g Aerosil 200 aufgestreut und durch gleichzeitiges Sprühen einer Lösung aus 10 Kollidon 25 in 500 g demineralisiertem Wasser an das Kernmaterial gebunden.
Auf 600 g der so hergestellten Theophyllin Pellets mit retardiertem Modulator in der Kernschicht a) wird in einer Wirbelschichtanlage eine Sprühsuspension aus 400 g EUDRAGIT^{®} RS 30 D (entsprechend 120 g Polymer), 60 g Talkum, 24 g Triethylcitrat, 0,6 g Eisenoxid Gelb und 538,3 g demineralisiertem Wasser aufgetragen. Die aufgetragene Polymermenge entsprcht somit 20 % des Ausgangsmaterials.

Die unter Beispiel 13 hergestellten Pellets können in einem USP Dissolutionstester in einem PhEur Phosphatpuffer pH 6.8 hinsichtlich Wirkstoffabgabe untersucht werden. Dabei wird man folgendes Retardierungsprinzip feststellen können:
Der Wirkstoffe wird innerhalb eines Zeitraums von 10 Stunden freigegeben, wobei die anfängliche Freigabe sehr gering ist. Über den untersuchten Zeitraum ist eine kontinuierliche Beschleunigung der Freigabe zu beobachten.

## Patentansprüche

1. Mehrschichtige Arzneiform für die kontrollierte Wirkstofffreisetzung, enthaltend,
a) eine Kernschicht, enthaltend eine in Bezug auf die Wirkstoffabgabe modulatorisch wirkende Substanz, gegebenenfalls einen Kern und/oder einen Wirkstoff,
b) eine innere Kontrollschicht, die die Abgabe der modulatorisch wirkenden Substanz und des gegebenenfalls enthaltenen Wirkstoffs aus der Kernschicht beeinflusst, enthaltend pharmazeutisch verwendbare Polymere, Wachse, Harze und/oder Proteine,
c) eine Wirkstoffschicht, enthaltend einen pharmazeutischen Wirkstoff und gegebenenfalls eine modulatorisch wirkende Substanz,
d) eine äußere Kontrollschicht, enthaltend zu mindestens 60 Gew.-% ein oder eine Mischung aus mehreren (Meth)acrylatcopolymeren, aus 98 bis 85 Gew.-% C₁- bis C₄-Alkylestern der (Meth)acrytsäure und 2 bis 15 Gew.-% Methacrylatmonomeren mit einer quaternären Ammoniumgruppe im Alkylrest, und gegebenenfalls bis zu 40 Gew.-% weitere pharmazeutisch verwendbare Polymere,
wobei die Schichten zusätzlich und in an sich bekannter Weise pharmazeutisch übliche Hilfsstoffe enthalten können.

2. Mehrschichtige Arzneiform nach Anspruch 1, **dadurch gekennzeichnet, daß** die Kernschicht a) alternativ und im wesentlichen folgende Bestandteile enthält:
I. eine modulatorisch wirkende Substanz in Kristall-, Granulat-, oder Copräzipitatform,
II. eine modulatorisch wirkende Substanz und einen Wirkstoff, die in beliebiger Reihenfolge schichtweise aufeinander oder in Mischung vorliegen können,
III. einen neutralen Kern (Nonpareilles), beschichtet mit einer modulatorisch wirkenden Substanz,
IV. einen neutralen Kern (Nonpareilles), beschichtet mit einer modulatorisch wirkende Substanz und einem Wirkstoff, die in beliebiger Reihenfolge schichtweise aufeinander oder in Mischung vorliegen können.

3. Mehrschichtige Arzneiform nach Anspruch 1 oder 2, **dadurch gekennzeichnet, daß** innere Kontrollschicht aus einem Polymeren besteht, das wasserunlöslich oder in Wasser nur quellbar ist.

4. Mehrschichtige Arzneiform nach Anspruch 3, **dadurch gekennzeichnet**, man das Polymer auswählt aus:
Copolymere aus Methylmethacrylat und/oder Ethylacrylat und Methacrylsäure, Copolymere aus Methylmethacrylat, Methylacrylat und Methacrylsäure, Copolymere aus Methylmethacrylat, Butylmethacrylat und Dimethylethylmethacrylat, Copolymere aus Methylmethacrylat, Ethylacrylat und Trimethylammoniumethylmethacrylat, Copolymere aus Methylmethacrylat und Ethylacrylat, Copolymere aus Ethylacrylat, Methylacrylat, Butylmethacrylat und Methacrylsäure
Polyvinylpyrolidone (PVP), Polyvinylalkohole, Polyvinylalkohol-Polyethylenglycol-Graft-Copolymer (Kollicoat®), Stärke und deren Derivate, Polyvinylacetatphtalat (PVAP, Coateric®), Polyvinylacetat (PVAc, Kollicoat), Vinylacetat-Vinylpyrolidon-Copolymer (Kollidon® VA64), Vinylacetat : Crotonsäure-Copolymer 9:1 (VAC : CRA, Kollicoat® VAC), Polyethylenglykole mit einem Molekulargewicht über 1000 (g/mol), Chitosan, ein (Meth)acrylatcopolymer, bestehend aus 20 - 40 Gew.-% Methylmethacrylat und 60 bis 80 Gew.-% Methacrylsäure, eine vernetzte und/oder unvernetzte Polyacrylsäure, ein Na-Alginat, und/oder ein Pektin,
Cellulosen wie z. B. anionische Carboxymethylcellulose und deren Salze (CMC, Na-CMC, Ca-CMC, Blanose, Tylopur), Carboxymethylethylcellulose (CMEC, Duodcell®), Hydroxyethylcellulose (HEC, Klucel), Hydroxypropylcellulose (HPC), Hydroxypropylmethylcellulose (HPMC, Pharmacoat, Methocel, Sepifilm, Viscontran, Opadry,), Hydroxymrthylethylcellulose (HEMC), Ethylcellulose (EC, Ethocel^{®}, Aquacoat^{®}, Surelease^{®}), Methylcellulose (MC, Viscontran, Tylopur, Methocel), Celluloseester, Celluloseglycolat, Celluloseacetatphtalat (CAP, Cellulosi acetas, PhEur, Celluloseacetate-phtalate, NF, Aquateric®), Celluloseacetatsuccinat (CAS), Celluloseacetattrimeliat (CAT), Hydroxypropylmethylcellulosephtalat (HPMCP, HP50, HP55), Hydroxypropylmethylcelluloseacetatsuccinat (HPMCAS -LF, -MF, -HF).

5. Mehrschichtige Arzneiform nach Anspruch 1 oder 2, **dadurch gekennzeichnet, daß** innere Kontrollschicht aus einem Wachs, wie z. B. Carnaubawachs und/oder Bienenwachs besteht.

6. Mehrschichtige Arzneiform nach Anspruch 1 oder 2, **dadurch gekennzeichnet, daß**, die Matrix der inneren Kontrollschicht das Harz Shellack enthält

7. Mehrschichtige Arzneiform nach Anspruch 1 oder 2, **dadurch gekennzeichnet, daß** innere Kontrollschicht aus einem Protein, wie z. B. Albumin, Gelatine, Gluten, Kollagen und/oder Zein besteht.

8. Mehrschichtige Arzneiform nach einem oder mehreren der Ansprüche 1 bis 6, **dadurch gekennzeichnet, daß** die modulatorisch wirkende Substanz ein Molekulargewicht unter 500 aufweist in fester Form vorliegt und ionogen ist.

9. Mehrschichtige Arzneiform nach Anspruch 7, **dadurch gekennzeichnet, daß** die modulatorisch wirkende Substanz wasserlöslich ist.

10. Mehrschichtige Arzneiform nach Anspruch 7 oder 8, **dadurch gekennzeichnet, daß**, die modulatorisch wirkende Substanz eine organische Säure oder das Salz einer organischen oder anorganischen Säure ist.

11. Mehrschichtige Arzneiform nach einem oder mehreren der Ansprüche 1 bis 9, **dadurch gekennzeichnet, daß** die modulatorisch wirkende Substanz Bernsteinsäure, Citronensäure, Weinsäure, Laurylschwefelsäure, ein Salz dieser Säuren oder ein Salz aus folgenden Anionen: Taurochlolat und andere Cholate, Chloride, Acetate, Lactate, Phosphate und/oder Sulfate ist.

12. Mehrschichtige Arzneiform nach einem oder mehreren der Ansprüche 1 bis 10, **dadurch gekennzeichnet, daß** die Wirkstoffschicht c) Metoprolol-Succinat enthält und die Wirkstofffreisetzung gemessen nach USP, 100 Upm, pH 6,8 in den 2 Stunden-Intervallen bis zur vierten Stunde langsamer als in den 2 Stunden-Intervallen von der vierten bis zur zehnten Stunde erfolgt.

13. Mehrschichtige Arzneiform nach einem oder mehreren der Ansprüche 1 bis 10, **dadurch gekennzeichnet, daß** die Wirkstoffschicht c) Terbutalin-Sulfat enthält und die Wirkstofffreisetzung gemessen nach USP, 100 Upm, pH 6,8 in 2 Stunden-Intervallen bis zur zwölften Stunde annähernd konstant erfolgt.

14. Verfahren zur Herstellung einer mehrschichtigen Arzneiform nach einem oder mehreren der Ansprüche 1 bis 12 in an sich bekannter Weise mittels pharmazeutisch üblicher Verfahren, wie direktem Verpressen, Verpressen von Trocken-, Feucht- oder Sintergranulaten, Extrusion und anschließende Ausrundung, feuchte oder trockene Granulation oder direkte Pelletierung oder durch Binden von Pulvern (Powder layering) auf wirkstofffreie Kugeln bzw. neutrale Kerne (Nonpareilles) oder wirkstoffhaltige Partikeln oder mittels Sprühverfahren oder Wirbelschichtgranulation.

15. Verwendung einer mehrschichtige Arzneiform nach einem oder mehreren der Ansprüche 1 bis 12 als Bestandteil einer multipartikulären Arzneiform, von pellethaltigen Tabletten, Minitabletten, Kapseln, Sachets, Brausetabletten oder Trockensäften.

## Claims

1. Multilayer pharmaceutical form for controlled active ingredient release, comprising
a) a core layer comprising a substance having a modulating effect in relation to active ingredient delivery, where appropriate a core and/or an active ingredient,
b) an inner controlling layer which influences the delivery of the substance having a modulating effect and of the active ingredient which is present where appropriate from the core layer, comprising pharmaceutically usable polymers, waxes, resins and/or proteins,
c) an active ingredient layer comprising an active pharmaceutical ingredient and, where appropriate, a substance having a modulating effect,
d) an outer controlling layer comprising at least 60% by weight of one or a mixture of a plurality of (meth)acrylate copolymers composed of 98 to 85% by weight C₁ to C₄ alkyl esters of (meth)acrylic acid and 2 to 15% by weight of methacrylate monomers with a quaternary ammonium group in the alkyl radical, and, where appropriate, up to 40% by weight of further pharmaceutically usable polymers,
where the layers may additionally and in a manner known per se comprise pharmaceutically usual excipients.

2. Multilayer pharmaceutical form according to Claim 1, **characterized in that** the core layer a) alternatively and essentially comprises the following ingredients:
I. a substance having a modulating effect, in crystalline, granular or coprecipitate form,
II. a substance having a modulating effect and an active ingredient, which may be present in successive layers in any sequence or in a mixture,
III. a neutral core (nonpareilles) coated with a substance having a modulating effect,
IV. a neutral core (nonpareilles) coated with a substance having a modulating effect and with an active ingredient, which may be present in successive layers in any sequence or in a mixture.

3. Multilayer pharmaceutical form according to Claim 1 or 2, **characterized in that** the inner controlling layer consists of a polymer which is insoluble in water or only swellable in water.

4. Multilayer pharmaceutical form according to Claim 3, **characterized in that** the polymer is selected from:
copolymers of methyl methacrylate and/or ethyl acrylate and methacrylic acid, copolymers of methyl methacrylate, methyl acrylate and methacrylic acid, copolymers of methyl methacrylate, butyl methacrylate and dimethylethyl methacrylate, copolymers of methyl methacrylate, ethyl acrylate and trimethylammoniumethyl methacrylate, copolymers of methyl methacrylate and ethyl acrylate, copolymers of ethyl acrylate, methyl acrylate, butyl methacrylate and methacrylic acid,
polyvinylpyrrolidones (PVPs), polyvinyl alcohols, polyvinyl alcohol-polyethylene glycol graft copolymer (Kollicoat®), starch and derivatives thereof, polyvinyl acetate phthalate (PVAP, Coateric®), polyvinyl acetate (PVAc, Kollicoat), vinyl acetate/vinylpyrrolidone copolymer (Kollidon® VA64), vinyl acetate: crotonic acid 9:1 copolymer (VAC: CRA, Kollicoat® VAC), polyethylene glycols with a molecular weight above 1000 (g/mol), chitosan, a (meth)acrylate copolymer consisting of 20-40% by weight of methyl methacrylate and 60 to 80% by weight of methacrylic acid, a crosslinked and/or uncrosslinked polyacrylic acid, an Na alginate, and/or a pectin,
celluloses such as, for example, anionic carboxymethylcellulose and salts thereof (CMC, Na-CMC, Ca-CMC, Blanose, Tylopur), carboxymethylethylcellulose (CMEC, Duodcell®), hydroxyethylcellulose (HEC, Klucel), hydroxypropylcellulose (HPC), hydroxypropylmethylcellulose (HPMC, Pharmacoat, Methocel, Sepifilm, Viscontran, Opadry), hydroxyethylmethylcellulose (HEMC), ethylcellulose (EC, Ethocel®, Aquacoat®, Surelease®), methylcellulose (MC, Viscontran, Tylopur, Methocel), cellulose esters, cellulose glycolate, cellulose acetate phthalate (CAP, Cellulosi acetas, PhEur, cellulose acetate phthalate, NF, Aquateric®), cellulose acetate succinate (CAS), cellulose acetate trimellitate (CAT), hydroxypropylmethylcellulose phthalate (HPMCP, HP50, HP55), hydroxypropylmethylcellulose acetate succinate (HPMCAS-LF, -MF, -HF).

5. Multilayer pharmaceutical form according to Claim 1 or 2, **characterized in that** the inner controlling layer consists of a wax such as, for example, carnauba wax and/or beeswax.

6. Multilayer pharmaceutical form according to Claim 1 or 2, **characterized in that** the matrix of the inner controlling layer comprises the resin shellac.

7. Multilayer pharmaceutical form according to Claim 1 or 2, **characterized in that** the inner controlling layer consists of a protein such as, for example, albumin, gelatin, gluten, collagen and/or zein.

8. Multilayer pharmaceutical form according to one or more of Claims 1 to 6, **characterized in that** the substance having a modulating effect has a molecular weight below 500 and is in solid form and is ionogenic.

9. Multilayer pharmaceutical form according to Claim 7, **characterized in that** substance having a modulating effect is soluble in water.

10. Multilayer pharmaceutical form according to Claim 7 or 8, **characterized in that** the substance having a modulating effect is an organic acid or the salt of an organic or inorganic acid.

11. Multilayer pharmaceutical form according to one or more of Claims 1 to 9, **characterized in that** the substance having a modulating effect is succinic acid, citric acid, tartaric acid, laurylsulphuric acid, a salt of these acids or a salt of the following anions: taurocholate and other cholates, chlorides, acetates, lactates, phosphates and/or sulphates.

12. Multilayer pharmaceutical form according to one or more of Claims 1 to 10, **characterized in that** the active ingredient layer c) comprises metoprolol succinate, and the active ingredient release measured according to USP, 100 rpm, pH 6.8, is slower in the 2-hour intervals up to the fourth hour than in the 2-hour intervals from the fourth to the tenth hour.

13. Multilayer pharmaceutical form according to one or more of Claims 1 to 10, **characterized in that** the active ingredient layer c) comprises terbutaline sulphate, and the active ingredient release measured according to USP, 100 rpm, pH 6.8 is approximately constant in 2-hour intervals up to the twelfth hour.

14. Process for producing a multilayer pharmaceutical form according to one or more of Claims 1 to 12 in a manner known per se by means of pharmaceutically customary processes such as direct compression, compression of dry, wet or sintered granules, extrusion and subsequent rounding off, wet or dry granulation or direct pelleting or by binding of powders (powder layering) onto active ingredient-free beads or neutral cores (nonpareilles) or active ingredient-containing particles or by means of spraying processes or fluidized bed granulation.

15. Use of a multilayer pharmaceutical form according to one or more of Claims 1 to 12 as ingredient of a multiparticulate pharmaceutical form, of pellet-containing tablets, minitablets, capsules, sachets, effervescent tablets or powders for reconstitution.

## Revendications

1. Forme de médicament multicouche pour la libération contrôlée de principes actifs, contenant :
a) une couche de noyau contenant une substance à action modulante sur base de la libération du principe actif, éventuellement un noyau et/ou un principe actif,
b) une couche interne de contrôle qui influence le dégagement de la substance à action modulante et du principe actif éventuellement contenu hors de la couche de noyau, contenant des cires, des résines, des protéines et/ou des polymères qu'on utilise en pharmacie,
c) une couche de principe actif contenant un principe actif pharmaceutique et éventuellement une substance à action modulante
d) une couche externe de contrôle contenant, pour au moins 60 % en poids un ou un mélange de plusieurs copolymères de (méth)acrylate, de 98 à 85 % en poids d'alkylesters en C₁-C₄ de l'acide (méth)acrylique et de 2 à 15 % en poids de monomères de méthacrylate avec un groupe ammonium quaternaire dans le radical alkyle et éventuellement jusqu'à 40 % en poids d'autres polymères utilisables en pharmacie,
les couches pouvant contenir en plus des adjuvants habituels en pharmacie d'une manière connue en soi.

2. Forme de médicament multicouche selon la revendication 1, **caractérisée en ce que** la couche de noyau a) contient en variante et essentiellement les constituants suivants :
I. une substance à action modulante sous forme de cristal, de granulé ou de coprécipitat,
II. une substance à action modulante et un principe actif qui peuvent être présents en couches successives dans un ordre quelconque ou en mélange,
III.un noyau neutre (nonpareilles), enrobé d'une substance à action modulante,
IV. un noyau neutre (nonpareilles), enrobé d'une substance à action modulante et d'un principe actif qui peuvent être présents en couches successives dans un ordre quelconque ou en mélange.

3. Forme de médicament multicouche selon la revendication 1 ou 2, **caractérisée en ce que** la couche interne de contrôle est constituée d'un polymère insoluble dans l'eau ou qui est seulement expansible dans l'eau.

4. Forme de médicament multicouche selon la revendication 3, **caractérisée en ce que** le polymère est choisi parmi les suivants :
des copolymères de méthylméthacrylate et/ou éthylacrylate et acide méthacrylique, des copolymères de méthylméthacrylate, méthylacrylate et acide méthacrylique, des copolymères de méthylméthacrylate, butylméthacrylate et diméthyléthylméthacrylate, des copolymères de méthylméthacrylate, éthylacrylate et triméthylammoniuméthylméthacrylate, des copolymères de méthylméthacrylate et éthylacrylate, des copolymères d'éthylacrylate, méthylacrylate, butylméthacrylate et d'acide méthacrylique
des polyvinylpyrrolidones (PVP), polyvinylalcools, copolymère greffé polyvinylalcool-polyéthylèneglycol (Kollicoat®), de l'amidon et ses dérivés, du polyvinylacétatephtalate (PVAP, Coateric®), du polyvinylacétate (PVAc, Kollicoat), copolymère vinylacétate-vinylpyrrolidone (Kollidon® VA64), vinylacétate: Copolymère acide crotonique 9:1 (VAC:CRA, Kollicoat® VAC), des polyéthylèneglycols avec un poids moléculaire supérieur à 1000 (g/mole), du chitosan, un copolymère de (méth)acrylate constitué de 20-40 % en poids de méthylméthacrylate et de 60 à 80 % en poids d'acide méthacrylique, un acide polyacrylique réticulé et/ou non réticulé, un alginate de Na et/ou une pectine,
des celluloses comme par exemple les suivants : carboxyméthylcellulose anionique et ses sels (CMC, Na-CMC, Ca-CMC, Blanose, Tylopur) carboxyméthyléthylcellulose (CMEC, Duodcell®), hydroxyéthylcellulose (HEC, Klucel), hydroxypropylcellulose (HPC), hydroxypropylméthylcellulose (HPMC, Pharmacoat, Méthocel, Sepifilm, Viscontran, Opadry,), hydroxyméthyléthylcellulose (HEMC), éthylcellulose (EC, Éthocel®, Aquacoat®, Surelease®), méthylcellulose (MC, Viscontran, Tylopur, Méthocel), esters de cellulose, glycolate de cellulose, acétate phtalate de cellulose (CAP, Cellulosi acetas, PhEur, acétate phtalate de cellulose, NF, Aquateric®), acétate succinate de cellulose (CAS), acétate trimellate de cellulose (CAT), phtalate d'hydroxypropylméthylcellulose (HPMCP, HP50, HP55), acétate succinate d'hydroxypropylméthylcellulose (HPMCAS -LF, -MF, -HF).

5. Forme de médicament multicouche selon la revendication 1 ou 2, **caractérisée en ce que** la couche interne de contrôle est constituée d'une cire, par exemple la cire de carnauba et/ou la cire d'abeilles.

6. Forme de médicament multicouche selon la revendication 1 ou 2, **caractérisée en ce que** la matrice de la couche de contrôle interne contient la résine Shellack.

7. Forme de médicament multicouche selon la revendication 1 ou 2, **caractérisée en ce que** la couche interne de contrôle est constituée d'une protéine, comme par exemple l'albumine, la gélatine, le gluten, le collagène et/ou la zéine.

8. Forme de médicament multicouche selon l'une ou plusieurs des revendications 1 à 6, **caractérisée en ce que** la substance à action modulante présente un poids moléculaire inférieur à 500, est présente sous forme solide et est ionogène.

9. Forme de médicament multicouche selon la revendication 7, **caractérisée en ce que** la substance à action modulante est soluble dans l'eau.

10. Forme de médicament multicouche selon la revendication 7 ou 8, **caractérisée en ce que** la substance à action modulante est un acide organique ou le sel d'un acide organique ou inorganique.

11. Forme de médicament multicouche selon l'une ou plusieurs des revendications 1 à 9, **caractérisée en ce que** la substance à action modulante est l'acide succinique, l'acide citrique, l'acide tartrique, l'acide laurylsulfurique, un sel de ces acides ou un sel des anions suivants: taurochlolate et d'autres cholates, des chlorures, des acétates, des lactates, des phosphates et/ou des sulfates.

12. Forme de médicament multicouche selon l'une ou plusieurs des revendications 1 à 10, **caractérisée en ce que** la couche de principe actif c) contient du métoprolol-succinate et la libération du principe actif, mesurée selon USP, 100 tr/min, pH 6,8, se fait dans des intervalles de deux heures jusqu'à la quatrième heure plus lentement que dans les intervalles de deux heures de la quatrième à la dixième heure.

13. Forme de médicament multicouche selon l'une ou plusieurs des revendications 1 à 10, **caractérisée en ce que** la couche de principe actif c) contient du terbutaline sulfate et la libération du principe actif, mesurée selon USP, 100 tr/min, pH 6,8, s'effectue dans des intervalles de deux heures jusqu'à la douzième heure de manière presque constante.

14. Procédé de fabrication d'une forme de médicament multicouche selon l'une ou plusieurs des revendications 1 à 12 d'une manière connue en soi au moyen de procédés habituels en pharmacie, comme la compression directe, la compression de granulés secs, humides ou frittés, l'extrusion avec arrondissage consécutif, la granulation au mouillé ou à sec ou la mise en pellets directe ou par liage de poudres (Powder layering) sur des billes sans principe actif ou, selon le cas, des noyaux neutres (Nonpareilles) ou des particules contenant le principe actif ou au moyen d'un procédé par pulvérisation ou granulation en lit fluidisé.

15. Utilisation d'une forme de médicament multicouche selon l'une ou plusieurs des revendications 1 à 12 comme constituant d'une forme de médicament multiparticulaire, de comprimés contenant les granulés, de minicomprimés, de capsules, de sachets, de comprimés effervescents ou de sirops secs.
